# EUROPEAN PATENT SPECIFICATION

(11) **EP 3 409 295 B1**
(45) Date of publication and mention of the grant of the patent: **30.12.2020**
(21) Application number: 17743661.5
(22) Date of filing: 20.01.2017
(51) Int. Cl.: A61K 47/60, A61P 25/04, A61P 25/36, A61K 47/55

(54) **PEGYLATED OPIOID WITH LOW ADDICTIVE EFFECT**
PEGYLIERTES OPIOID MIT GERINGER SUCHTWIRKUNG
OPIOÏDES PÉGYLÉS À FAIBLE EFFET ADDICTIF

(30) Priority: 29.01.2016 CN 201610067072
(43) Date of publication of application: 05.12.2018
(73) Proprietor: Jenkem Technology Co., Ltd. (Beijing), Haidian District Beijing 100192 (CN)
(72) Inventor: FENG, Zewang, Beijing 100192 (CN); WANG, Jinliang, Beijing 100192 (CN); LIU, Yan, Beijing 100192 (CN); ZHAO, Xuan, Beijing 100192 (CN)
(74) Representative: ABG Intellectual Property Law, S.L.
(86) International application number: PCT/CN2017/071797
(87) International publication number: WO 2017/129046

(56) References cited:
- EP-A1- 2 818 169
- WO-A1-2011/011543
- CN-A- 1 995 094
- CN-A- 101 724 144
- CN-A- 102 159 249
- CN-A- 102 711 837
- CN-A- 104 292 454
- CN-A- 104 371 098
- CN-A- 104 448 296
- CN-A- 104 473 927
- CN-A- 104 473 927
- FRENSCH K ET AL: "OLIGO ETHYLENE GLYCOL ETHERS OF MORPHINE", LIEBIGS ANNALEN DER CHEMIE, VERLAG CHEMIE GMBH. WEINHEIM, DE, no. 12, 1 January 1979 (1979-01-01), pages 2118-2120, XP002544616, ISSN: 0170-2041, DOI: 10.1002/JLAC.197919791222

## Description

### FIELD OF THE INVENTION

The present invention relates to a conjugate of a hydrophilic polymer and an opioid, especially a pegylated opioid with low addictive effect and a pharmaceutical composition thereof, as defined in the claims.

### BACKGROUND OF THE INVENTION

Opioids (also known as opioid receptor agonists, such as morphine, codeine, etc.) have an extremely strong analgesic effect and have been widely used as analgesics in clinics, which interact with opioid receptors (including µ, κ and δ receptors, especially µ and κ receptors) to produce analgesic effects.

However, opioids may cause drug resistance and physiological dependence (i.e., addiction), which is the main problem in use of opioids for analgesia, while relieving nerve pain with repeated administration. The euphoric effect generated during medication, and discomfort caused by discontinuation of medication (such as anorexia, weight loss, anxiety, irritability, dizziness, abdominal pain, emesis, spasm, running nose, and tachycardia, etc.) force patients to seek medications. The factors, such as possible side effects caused by a dose beyond the normal dose range, possible flow of the drugs from pain sufferers to addicts, etc., lead to a potential abuse of the opioids in the application.

At present, one way to prevent the addiction of an opioid drug is to combine the opioid with an opioid antagonist to block the euphoric effect through the opioid antagonist. For example, in the prior art, a compound tablet of pentazocine and naloxone (e.g., Talwin Nx, pentazocine hydrochloride (50 mg) and naloxone hydrochloride (50 mg)) is used to relieve pain, the content of naloxone in the compound tablet makes it less active and hardly interfere with the pharmacological action of pentazocine in oral administration, but may obviously antagonize the analgesic effect in parenteral administration, and a soluble or injectable formulation may limit the possible abuse of pentazocine; in addition, a compound drug of tilidine (50 mg) and naloxone (50 mg) is also used to treat severe pain, which utilizes antagonism of naloxone against morphine receptors to avoid addiction to tilidine. Although the opioids and the opioid antagonists have been used in combination in the prior art, under the conditions that the tablets are crushed or chopped, the opioid antagonists have increased solubility, antagonize the opioid receptors, and prevent the drug from being abused parenterally, opioids without antagonists may also be extracted if the tablets are carefully dissolved (e.g., placing the tablets in water overnight) without need to be smashed, resulting in abuse. In addition, opioid antagonists have a high blood-brain barrier transmission rate, and may counteract the analgesic effects of opioids while reducing the addiction and discomfort caused by long-term use of opioids in patients with chronic pain.

In the prior art, opioids have also been covalently bound to a water-soluble oligomer to change the pharmacokinetic profile of opioids, which may deliver opioids to the brain at a constant low concentration, avoiding the peak concentration (which is the basis for the addiction potential) of traditional delivery methods to achieve reduced addiction and prevent drug abuse and addiction. Specifically, prior art CN200980136089.1 discloses mPEGₙ-O-morphine, mPEGₙ-O-hydrocodone, and mPEGₙ-O-codeine conjugates, by linking polyethylene glycol to the opioids described above, reduced drug addiction can be achieved while retaining the analgesic effects of the drugs. In the above conjugates, the polyethylene glycol is linked with only one active component. Although the above opioid-polyethylene glycol conjugates have reduced drug addiction, they have a significant decrease in the affinity between the opioids and the opioid receptors as the polymer molecular weight increases.

In order to overcome the defects of the prior art, the present invention realized the linkage of polyethylene glycol and a plurality of opioids, and screened several conjugates of polyethylene glycol and opioids with low addiction. Different from the prior art, while having low addiction and potential for abuse, the conjugate of the present invention has a superior activity with the opioid receptors over a single-opioid conjugate with the same molecular weight, has a more improved pharmacokinetic profile, and can achieve administration with a smaller dose in clinical practice WO2011/011543 discloses mPEG-morphine, mPEG-codeine, mPEG-hydroxycodone and mPEG-fentanyl, but does not describe that 2-6 opioid units are linked via a PEG linker.

### SUMMARY OF THE INVENTION

The invention is defined by the claims. Any subject-matter falling outside the scope of the claims is provided for information purposes only.

An object of the present invention is to provide a conjugate of polyethylene glycol and di-opioid with low addictive effect.

Another object of the present invention is to provide a pharmaceutical composition comprising the conjugate of the present invention and a pharmaceutically acceptable carrier or excipient thereof.

Still another object of the present invention is to provide an application of the conjugate of the present invention and a pharmaceutical composition thereof in the preparation of an analgesic drug.

In order to realize the objects of the present invention, the present invention adopts the following technical solution:
In one aspect, the present invention provides a conjugate of polyethylene glycol and opioid having a structure of formula (I):

PEG- (X-OP)ₘ (I)

wherein,
PEG is a double-ended, branched or multi-armed polyethylene glycol residue with a molecular weight of 45-1500 Da;
X is a linking group selected from one of the following group: -O-, -OC(O)-, -O(CH₂)ᵢNH-, -O(CH₂)ᵢC(O)O-, -O(CH₂)ᵢC(O)NH-, and -O(CH₂)ᵢNHC(O)NH-, i is an integer of 0-5, preferably 0, 1, or 2;
OP is opioid; and
m is an integer of 2-6, preferably 2, 3 or 4.

In an embodiment of the present invention, the opioid has a structure of the following formula (II-1) or (II-2): 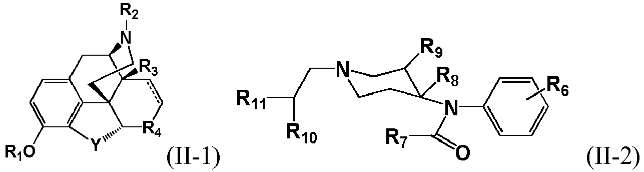 wherein:
R₁ is -H, methyl, ethyl, or -C(O)CH₃;
R₂ is -H, methyl, ethyl, isopropyl, propenyl, methylcyclopropane, or methylcyclobutane;
R₃ is -H or -OH;
R₄ is 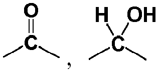 or 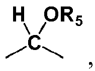 R₅ is -H, methyl, ethyl or -C(O)CH₃; the dashed line is an optional double bond;
Y is O or S;
R₆ is -H, methyl or methoxy;
R₇ is -H, methyl, ethyl, cyclopropyl, methoxy, ethoxy, or dimethylamino;
R₈ is -H, -CH₂OCH₃, -C(O)OCH₃, or -C(O)CH₃;
R₉ is -H or methyl;
R₁₀ is -H or -OH; and
R₁₁ is phenyl, hydroxyphenyl, cyclohexyl, halophenyl, methoxyphenyl, 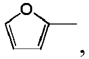 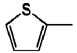 or -C(O)OCH₃. Wherein, the halophenyl refers to the phenyl ring that is substituted at one or more available positions by one or more halo groups, and the halo refers to bromo, chloro, iodo or fluoro.

In an embodiment of the present invention, the opioid may specifically be: morphine, hydromorphone, oxycodone, hydrocodone, codeine, paracodin, nalbuphine, methofuran, levorphan, levorphanol, butorphanol, oxymorphone, buprenorphine, fentanyl and a pharmaceutically acceptable salt thereof; preferably, the opioid is morphine, oxycodone, codeine or fentanyl.

In an embodiment of the present invention, the "PEG" or "polyethylene glycol" includes any water-soluble poly(ethylene oxide); the molecular weight of the PEG is between about 45 and 1500 Da, e.g., between 45 and 450 Da (including 45-90 Da, 45-135 Da, 90-135 Da, 90-180 Da, 90-225 Da, 90-270 Da, 135-225 Da, 135-270 Da, 225-315 Da, 315-360 Da, 360-405 Da, 405-450 Da), between 450 and 900 Da (including 450-500 Da, 500-550 Da, 550-600 Da, 600-650 Da, 650-700 Da, 700-750 Da, 750-800 Da, 800-850 Da, 850-900 Da), or between 900 and 1500 Da (including 900-1500 Da, 900-1350 Da, 900-1200 Da, 900-1050 Da), preferably, the molecular weight is less than 450 Da; and the PEG may be a double-ended, branched or multi-armed polyethylene glycol residue.

In an embodiment of the present invention, the linking group X links the PEG with the opioid in a covalent bond form, the covalent bond being substantially stable in water and exhibiting a hydrolysis rate of less than about 2%/day under normal physiological conditions. The present invention links the polyethylene glycol with the opioid via the linking group in a manner known in the art. Illustratively, a terminal group of the polyethylene glycol is modified with an electrophilic group that reacts with a nucleophilic group (such as hydroxyl, amino, etc.) in the opioid to form a covalent linkage. Under the condition that the opioid itself does not contain a nucleophilic group, the group that needs to be linked can be modified so that the reaction can occur (e.g., the ketone group is reduced to a hydroxyl group). Under the condition that the opioid contains a group that is not desired to react, it can be protected by a protective group (e.g., the hydroxyl group that is not desired to react may be protected using MEMCI) and then subjected to a coupling reaction, and deprotected after the completion of the reaction.

Preferably, the linking group X is -O-, the conjugate has a structure of formula (I-0): PEG-(O-OP)ₘ (I-0).

In a specific embodiment of the present invention, the PEG is a double-ended polyethylene glycol residue, m is 2, and the conjugate of formula (I) is a conjugate of polyethylene glycol and double opioid having a structure of the following formula (III):

OP-X-PEG-X-OP (III)

wherein,
PEG is a polyethylene glycol residue of the following formula: -(CH₂CH₂O)ₙ₋₁CH₂CH₂-, n is an integer of 1-30;
X is a linking group selected from one of the following group: -O-, -OC(O)-, -O(CH₂)ᵢNH-, -O(CH₂)ᵢC(O)O-, -O(CH₂)ᵢC(O)NH-, and -O(CH₂)ᵢNHC(O)NH-, i is an integer of 0-5, preferably 0, 1, or 2;
OP is an opioid.

In a specific embodiment of the present invention, the polyethylene glycol residue of the formula (III) may be: -(CH₂CH₂O)ₙ₋₁CH₂CH₂-, which may also be described as -(CH₂CH₂O)ₙ- in certain embodiments, and n is preferably an integer of 2-20 (specifically 2, 3, 4, 5, 6, 7, 8, 9, 10, 11, 12, 13, 14, 15, 16, 17, 18, 19, or 20), more preferably, n is an integer of 2-10 (specifically 2, 3, 4, 5, 6, 7, 8, 9, or 10), and most preferably, n is an integer of 3-7 (specifically 3, 4, 5, 6, or 7).

In a specific embodiment of the present invention, X is -O-, and the formula (III) has a structure of the following formula (III-0):

OP-O-PEG-O-OP (III-0).

Specifically, the formula (III) may be a structure represented by the following formula (III-1), (III-2) or (III-3):

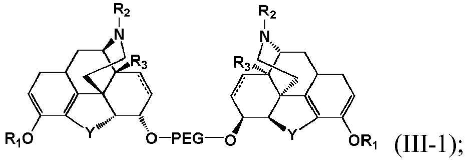

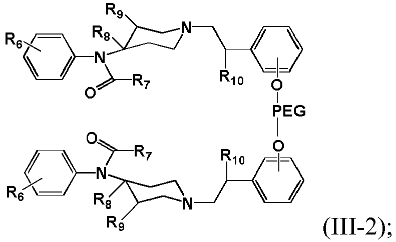

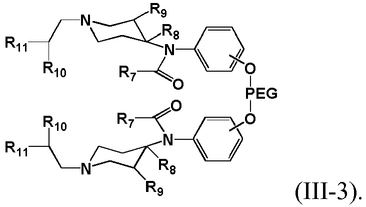

In a specific embodiment of the present invention, the formula (III) has a structure of the following formula (IV-1): 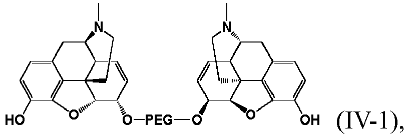 wherein, the PEG is a double-ended polyethylene glycol residue: -(CH₂CH₂O)ₙ₋₁CH₂CH₂-, n is an integer of 2-10, preferably 3, 4, 5, 6, or 7.

In a specific embodiment of the present invention, the formula (III) has a structure of the following formula (IV-2): 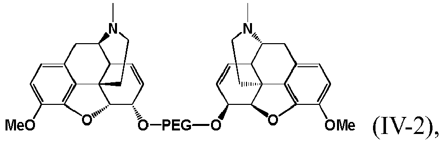 wherein, the PEG is a double-ended polyethylene glycol residue: -(CH₂CH₂O)ₙ₋₁CH₂CH₂-, n is an integer of 2-10, preferably 3, 4, 5, 6, or 7.

In a specific embodiment of the present invention, the formula (III) has a structure of the following formula (IV-3): 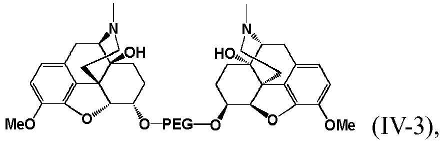 wherein, the PEG is a double-ended polyethylene glycol residue: -(CH₂CH₂O)ₙ₋₁CH₂CH₂-, n is an integer of 2-10, preferably 3, 4, 5, 6, or 7.

In a specific embodiment of the present invention, the formula (III) has a structure of the following formula (IV-4): 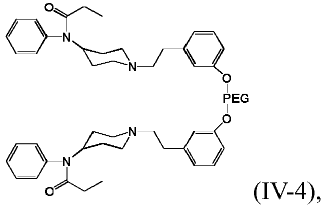 wherein, the PEG is a double-ended polyethylene glycol residue: -(CH₂CH₂O)ₙ₋₁CH₂CH₂-, n is an integer of 2-10, preferably 3, 4, 5, 6, or 7.

In a specific embodiment of the present invention, the formula (III) has a structure of the following formula (IV-5): 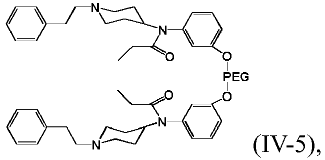 wherein, the PEG is a double-ended polyethylene glycol residue: -(CH₂CH₂O)ₙ₋₁CH₂CH₂-, n is an integer of 2-10, preferably 3, 4, 5, 6, or 7.

In a specific embodiment of the present invention, the formula (III) has a structure of the following formula (IV-6): 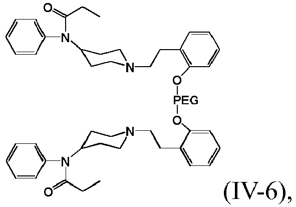 wherein, the PEG is a double-ended polyethylene glycol residue: -(CH₂CH₂O)ₙ₋₁CH₂CH₂-, n is an integer of 2-10, preferably 3, 4, 5, 6, or 7.

In a specific embodiment of the present invention, the formula (III) has a structure of the following formula (IV-7): 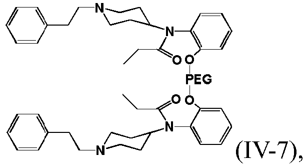 wherein, the PEG is a double-ended polyethylene glycol residue: -(CH₂CH₂O)ₙ₋₁CH₂CH₂-, n is an integer of 2-10, preferably 3, 4, 5, 6, or 7.

In a specific embodiment of the present invention, the formula (III) has the following structure of formula (IV-8): 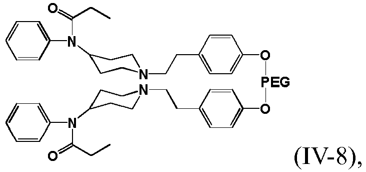 wherein, the PEG is a double-ended polyethylene glycol residue: -(CH₂CH₂O)ₙ₋₁CH₂CH₂-, n is an integer of 2-10, preferably 3, 4, 5, 6, or 7.

In a specific embodiment of the present invention, the formula (III) has the following structure of formula (IV-9): 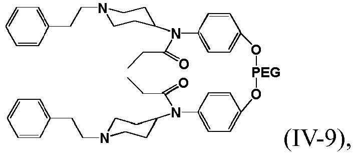 wherein, the PEG is a double-ended polyethylene glycol residue: -(CH₂CH₂O)ₙ₋₁CH₂CH₂-, n is an integer of 2-10, preferably 3, 4, 5, 6, or 7.

In a specific embodiment of the present invention, the PEG is a three-armed polyethylene glycol residue, m is 3, and the conjugate of the formula (I) is a conjugate of polyethylene glycol and tri-opioid with a structure of formula (V): 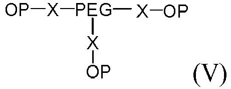 wherein, PEG has a branched polyethylene glycol residue of the following formula: 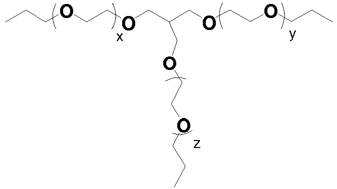
wherein, x, y, or z may be independently selected from an integer of 1-20, preferably, an integer of 1-10 (specifically 1, 2, 3, 4, 5, 6, 7, 8, 9, or 10), and more preferably an integer of 1-6 (specifically 1, 2, 3, 4, 5, or 6). Preferably, the sum of x, y and z is an integer less than 10, more preferably an integer less than 7, and most preferably an integer less than 5, for example, x, y and z are all 1;
X is a linking group selected from one of the following group: -O-, -OC(O)-, -O(CH₂)ᵢNH-, -O(CH₂)ᵢC(O)O-, -O(CH₂)ᵢC(O)NH-, and -O(CH₂)ᵢNHC(O)NH-, i is an integer of 0-5, preferably 0, 1, or 2;
OP is an opioid.

In a specific embodiment of the present invention, the X is -O-, and the formula (V) has a structure of the following formula (V-0):

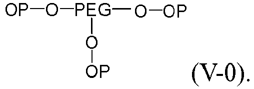

Specifically, the formula (V) may be a structure represented by the following formula (V-1), (V-2), or (V-3):

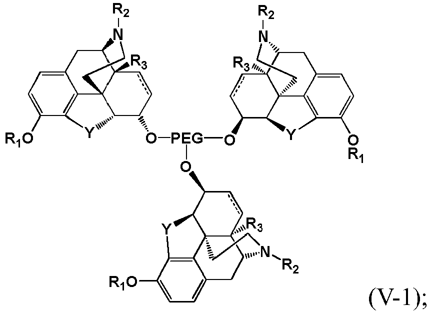

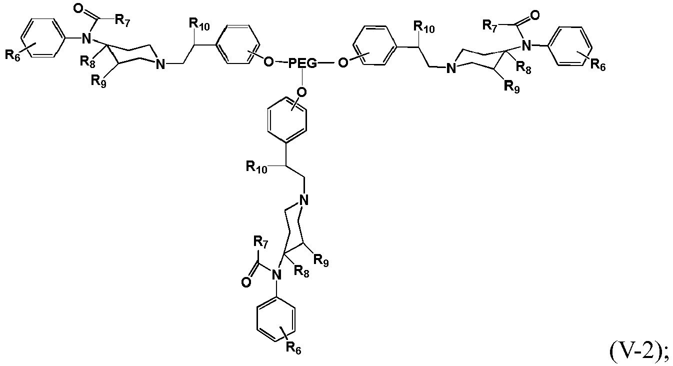

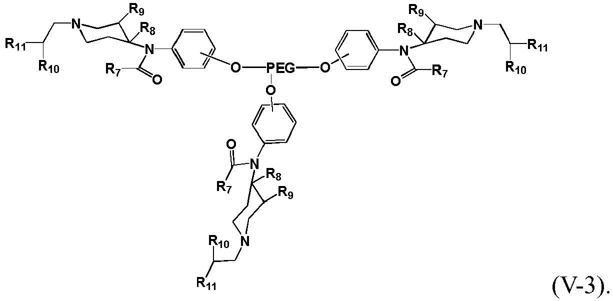

In a specific embodiment of the present invention, the PEG is a four-arm polyethylene glycol residue, the m is 4, and the conjugate of the formula (I) is a conjugate of polyethylene glycol and tetra-opioid having a structure of formula (VI): 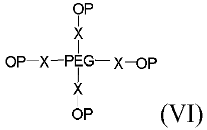 wherein, the PEG has a branched polyethylene glycol residue represented by the following formula: 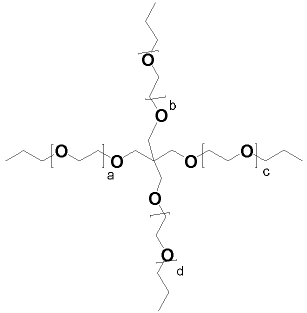
wherein, a, b, c or d may be independently selected from the integer of 1-20, preferably, an integer of 1-10 (specifically 1, 2, 3, 4, 5, 6, 7, 8, 9, or 10), and more preferably an integer of 1-6 (specifically 1, 2, 3, 4, 5, or 6). Preferably, the sum of a, b, c, and d is an integer less than 10, more preferably an integer less than 7, and most preferably an integer less than 5, for example, a, b, c, and d are all 1;
X is a linking group selected from one of the following group: -O-, -OC(O)-, -O(CH₂)ᵢNH-, -O(CH₂)ᵢC(O)O-, -O(CH₂)ᵢC(O)NH-, and -O(CH₂)ᵢNHC(O)NH-, i is an integer of 0-5, preferably 0, 1, or 2;
OP is an opioid.

In a specific embodiment of the present invention, the X is -O-, and the formula (VI) has a structure of the following formula (VI-0):

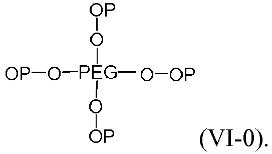

Specifically, the formula (VI) may be a structure represented by the following formula (VI-1), (VI-2) or (VI-3):

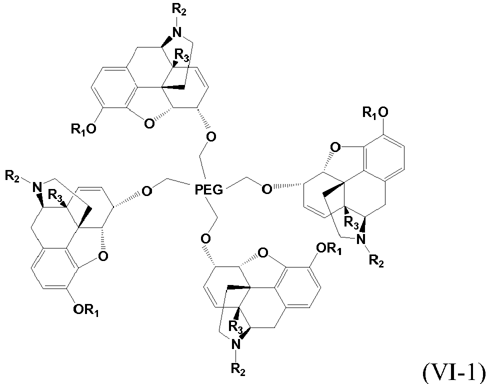

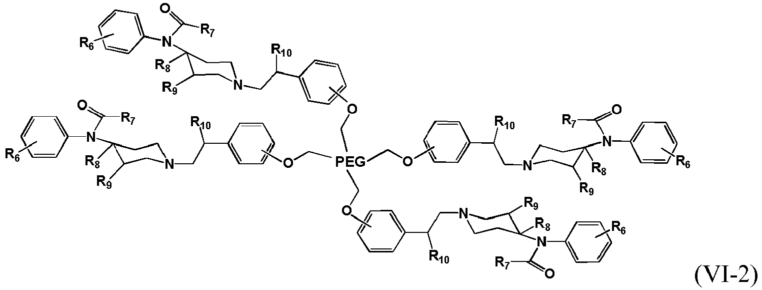

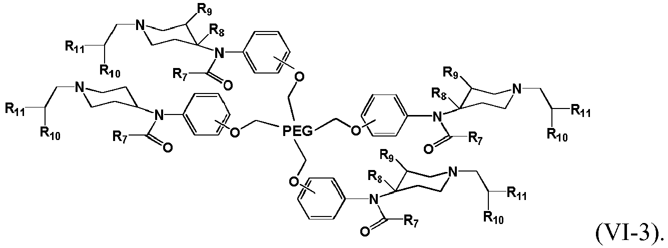

According to a further aspect of the present invention, a conjugate of formula (I) or a pharmaceutically acceptable salt thereof is provided.

The above pharmaceutically acceptable salt may be sodium salt, potassium salt, cesium salt, calcium salt, magnesium salt, triethylamine salt, pyridinium salt, picoline salt, ethanolamine salt, triethanolamine salt, dicyclohexylamine salt, N,N-dibenzylethylenediamine salt, hydrochloride, hydrobromide, sulfate, nitrate, phosphate, formate, acetate, trifluoroacetate, citrate, tartrate, fumarate, maleate, lactate, mesylate, benzene sulfonate, tosilate, arginine, aspartate, glutamate, pantothenate, ascorbate, etc., or a combination thereof.

Preferably, the pharmaceutically acceptable salt may be hydrochloride, hydrobromide, sulfate, nitrate, phosphate, citrate, tartrate, fumarate, maleate, lactate, benzene sulfonate, pantothenate, ascorbate, etc., or a combination thereof.

The present invention also provides a pharmaceutical composition comprising the conjugate or pharmaceutically acceptable salt thereof of the present invention and a pharmaceutically acceptable carrier or excipient.

In some embodiments, depending on the desired mode of administration, the pharmaceutical composition may comprise about 1 to about 99% by weight of the conjugate of the present invention, and 99 to 1% by weight of a suitable carrier or pharmaceutically acceptable excipient. Preferably, the composition comprises about 5 to 75% by weight of the conjugate of the present invention, with the balance being a suitable carrier or pharmaceutically acceptable excipient. More preferably, the composition comprises about 10 to 50% by weight of the conjugate of the invention, with the balance being a suitable carrier or pharmaceutically acceptable excipient.

In some embodiments, the pharmaceutical composition of the present invention may further comprise a small number of auxiliary substances such as wetting or emulsifying agents, pH buffers, antioxidants, and the like, e.g., citric acid, sorbitan monolaurate, triethanolamine oleate, butylated hydroxytoluene and the like.

In some embodiments, the pharmaceutical compositions are tablets, capsules, pills, granules, powders, suppositories, injections, solutions, suspensions, ointments, patches, lotions, drops, liniments, sprays, and the like.

In some embodiments, the conjugate of the present invention may be administered in the form of a pure compound or a suitable pharmaceutical composition, using any acceptable means of administration or reagents for a similar purpose. Thus, the mode of administration adopted may be oral, intranasal, parenteral, topical, transdermal or rectal, in the form of solid, semi-solid, or liquid agents, for example, tablets, suppositories, pills, soft and hard gelatin capsules, powders, solutions, suspensions and injections, etc., preferably a unit dosage form suitable for use in precise dosages for simple administration.

The pharmaceutical compositions that can be administered in liquid form are, for example, solutions or suspensions, which are formed by dissolving or dispersing the conjugates of the present invention (about 0.5 to about 20%) and optionally present pharmaceutically acceptable adjuvants in a carrier by dissolving, dispersing, and other methods, and the examples of the carrier are water, saline, glucose hydrate, glycerol, and ethanol, etc.

The present application also relates to an application of the conjugate, the pharmaceutically acceptable salt thereof, or the pharmaceutical composition of the present invention in the preparation of an analgesic drug, and the drug has a reduced addiction and abuse.

The conjugate of polyethylene glycol and polyopioids of the present invention has low addiction and potential for abuse while having a superior activity with opioid receptors over single-opioid conjugates with the same molecular weight, and has a more improved pharmacokinetic profile, and can achieve the administration with a smaller dose in the clinic.

### DETAILED DESCRIPTION OF THE INVENTION

The following examples are used to illustrate the present invention

The morphine and codeine used in the Examples were purchased from Qinghai Pharmaceutical Factory Co., Ltd.; oxycodone was purchased from Beijing Wellso Pharmaceutical Co., Ltd., methoxyethoxymethyl chloride (MEMCL) was purchased from Alfa Aesar, toluenesulfonyl chloride was purchased from Shandong Yilong Industrial Co., Ltd., sodium hydride was purchased from TCI (Shanghai) Development Co., Ltd., and H(OCH₂CH₂)₂-OH, H(OCH₂CH₂)₃-OH, H(OCH₂CH₂)₄-OH, H(OCH₂CH₂)₅-OH, H(OCH₂CH₂)₆-OH, H(OCH₂CH₂)₇-OH, H(OCH₂CH₂)₈-OH and H(OCH₂CH₂)₉-OH were purchased from Biomatrik Inc. The other reagents used in the Examples of the present invention are all Commercial chemically pure reagents.

### Example 1: Preparation of PEGₙ-(OMs)₂

To a 250 mL three-necked flask, diethylene glycol (10.0 g, 94.3 mmol) and dichloromethane (100 mL) were added. The mixture was stirred and cooled to 0°C. Triethylamine (19.1 g, 188.6 mmol) was added dropwise. The reaction mixture was stirred for 10 minutes. Methanesulfonyl chloride (21.6 g, 188.6 mmol) was added to the reaction mixture over 5-10 minutes and stirred at room temperature for 12 hours. The progress of the reaction was monitored by TLC. After the reaction was complete, distilled water was added to the reaction mixture, followed by extraction with dichloromethane (3×100 mL). The organic layers were combined, washed with distilled water (3×100 mL), dried over anhydrous sodium sulfate and concentrated to dryness under reduced pressure using a rotary evaporator to give 21.8 g of a brown oil, yield: 88.2%. ¹H NMR (400 MHz, CDCl₃): δ 4.10 (t, 4H), 3.86 (t, 4H), 3.19 (s, 6H).

Other MsO-PEGₙ-OMs (n=l, 3, 4, 5, 6, 7, 8, or 9) were prepared with the same method, and the products were confirmed by ¹H NMR and LC-MS.

### Example 2: Preparation of PEGₙ-(morphine)₂ Conjugate

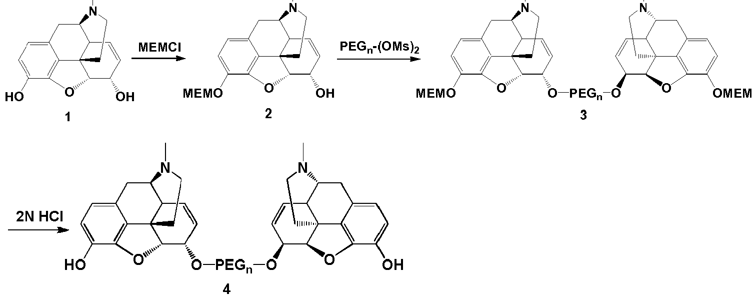

### Step 1: Preparation of 3-O-MEM- morphine (2)

Morphine sulphate (1.00 g, 2.64 mmol) was dissolved in acetone/toluene (70 mL/35 mL) at room temperature. The mixture was stirred uniformly. To the mixture was added K₂CO₃ (1.35 g, 9.77 mmol), stirred for 25 minutes, and then MEMCI (0.66 g, 5.28 mmol) was added. The reaction mixture was stirred at room temperature for 24 hours. Then anhydrous methanol (1.2 mL) was added to terminate the reaction. The reaction mixture was concentrated under reduced pressure to dryness. Distilled water (15 mL) and saturated brine (45 mL) were added to the residue, which was extracted with ethyl acetate (3×50 mL). The combined organic solution was washed with saturated brine (3×50 mL), dried over anhydrous sodium sulfate and concentrated under reduced pressure. The residue was purified using a Biotage flash purification preparative liquid chromatograph to give 0.52 g of a pale yellow oil, yield: 52.7%. ¹H NMR (400 MHz, CDCl₃): δ 6.46 (d, 1H), 6.33 (d, 1H), 6.02 (s, 2H), 5.59 (t, 2H), 4.30 (d, 1H), 4.19 (t, 1H)), 3.54(t, 4H), 3.32 (s, 1H), 3.24(s, 3H), 2.91(d, 2H), 2.76 (m, 1H), 2.49(m, 1H), 2.27(s, 3H), 2.24(m, 2H), 1.75 (m, 2H).

### Step 2: Preparation of 6-O-PEGₙ-(3-O-MEM-morphine)₂ (3)

To a mixed solution of toluene/DMF (8-fold/4-fold volume) was added 3-O-MEM-morphine (2.4- to 2.8-fold equivalent) followed by NaH (8- to 12-fold equiv) and then PEGₙ-(OMs)₂. The reaction mixture was heated to 55-75°C and incubated with stirring until the reaction was confirmed by LC-MS analysis to be complete (depending on the chain length of PEG, 12-40 hours). The reaction was quenched with anhydrous methanol (10-fold volume). The reaction mixture was concentrated under reduced pressure to dryness. The residue was purified using a Biotage flash purification preparative liquid chromatograph to give a yellow to orange oil, yield: 15-30%.

6-O-PEGₙ-(3-O-MEM-morphine)₂ (n = 1, 2, 3, 4, 5, 6, 7, 8, or 9) were prepared by this method, and the products were confirmed by ¹H NMR and LC-MS.

### Step 3: Preparation of 6-O-PEGₙ-(morphine)₂ Hydrochloride (4)

6-O-PEGₙ-(3-O-MEM-morphine)₂ was dissolved in dichloromethane (16-fold volume) and 2 M HCl in diethyl ether (12-foldvolume) was added. The reaction mixture was stirred at room temperature for 2 hours and evaporated under reduced pressure to remove the solvent. The residue was dissolved in anhydrous methanol (16-fold volume) and filtered. The filtrate was concentrated under reduced pressure to dryness to give a yellow to orange oil, yield: 95-100%, HPLC purity (UV254): 95-98%. The compounds prepared by this method include: α-6-O-PEG₁-(morphine)₂; α-6-O-PEG₂-(morphine)₂; α-6-O-PEG₃-(morphine)₂; α-6-O-PEG₄-(morphine)₂; α-6-O-PEG₅-(morphine)₂; α-6-O-PEG₆-(morphine)₂; α-6-O-PEG₇-(morphine)₂; α-6-O-PEG₈-(morphine)₂ and α-6-O-PEG₉-(morphine)₂. The products were confirmed by ¹H NMR and LC-MS.

### Example 3: Preparation of PEGₙ-(codeine)₂ Conjugate

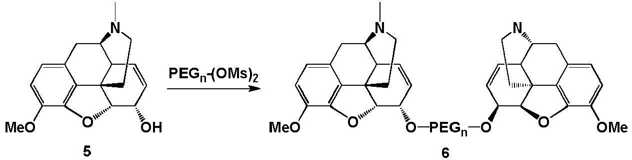

### Step 1: Preparation of 6-O-PEGₙ-(codeine)₂ Free Alkali (6)

To a mixed solution of toluene/DMF (24-fold/1-fold volume) was added codeine (2.4- to 2.8-fold equiv) followed by NaH (8- to 12-fold equiv) and then PEGₙ-(OMs)₂. The reaction mixture was heated to 45-65°C and incubated with stirring until the reaction was confirmed by LC-MS analysis to be complete (depending on the chain length of PEG, 12-48 hours). The reaction was quenched with anhydrous methanol (10-fold volume). The reaction mixture was concentrated under reduced pressure to dryness. The residue was purified using a Biotage flash purification preparative liquid chromatograph to give a yellow to orange oil, yield: 12-25%. 6-O-PEGₙ-(codeine)₂ free alkali (n=l, 2, 3, 4, 5, 6, 7, 8, or 9) were prepared by this method, and the products were confirmed by ¹H NMR and LC-MS.

### Step 2: Preparation of 6-O-PEGₙ-(codeine)₂ Hydrochloride

6-O-PEGₙ-(codeine)₂ free alkali was dissolved in dichloromethane (16-fold volume) and 2 M HCl in diethyl ether (12-fold volume) was added. The reaction mixture was stirred at room temperature for 2 hours and evaporated under reduced pressure to remove the solvent. The residue was dissolved in anhydrous methanol (16-fold volume) and filtered. The filtrate was concentrated under reduced pressure to dryness to give a yellow to orange oil, yield: 95-100%, HPLC purity (UV254): 95-98%. The compounds prepared by this method include: α-6-O-PEG₁-(codeine)₂; α-6-O-PEG₂-(codeine)₂; α-6-O-PEG₃-(codeine)₂; α-6-O-PEG₄-(codeine)₂; α-6-O-PEG₅-(codeine)₂; α-6-O-PEG₆-(codeine)₂; α-6-O-PEG₇-(codeine)₂; α-6-O-PEG₈-(codeine)₂ and α-6-O-PEG₉-(codeine)₂. The products were confirmed by ¹H NMR and LC-MS.

### Example 4: Preparation of PEGₙ-(oxycodol)₂ Conjugate

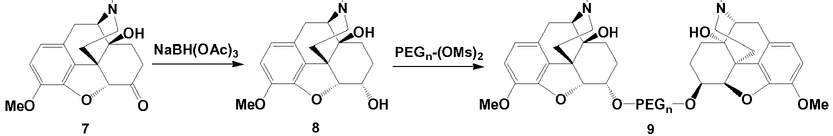

### Step 1: Preparation of oxycodol (8) by selective reduction of oxycodone

To a solution of oxycodone (3.79 g, 12.03 mmol) in anhydrous methanol (150 mL) was added NaBH(OAc)₃ (7.65 g, 36.09 mmol) and AcOH (2.17 g, 36.09 mmol) with stirring at 15°C. The reaction mixture was stirred at 15-20°C for 24 h and the progress of the reaction was monitored by TLC. The reaction mixture was evaporated under reduced pressure to remove the solvent. Methylene chloride (100 mL) was added to the residue. The pH of the solution was adjusted to 10 with saturated NaHCO₃ aqueous solution. Distilled water (100 mL) was added to the mixed solution. The mixture was extracted with dichloromethane (2×100 mL). The combined organic phase was washed with saturated brine (100 mL), dried over anhydrous sodium sulfate, and evaporated under reduced pressure to remove the solvent to give 3.72 g of a colorless oil, yield: 97.4%. ¹H NMR (400 MHz, CDCl₃): 6.46 (d, 1H), 6.35 (d, 1H), 4.10 (d, 1H), 3.84 (s, 3H), 3.67 (m, 2H), 3.59 (s, 1H), 3.07 (m, 1H), 2.79 (m, 2H), 2.49 (m, 2H), 2.27 (s, 3H), δ 1.45 - 1.92 (m, 6H).

### Step 2: Preparation of 6-O-PEGₙ-(oxycodol)₂ Free Alkali (9)

To a mixed solution of toluene/DMF (8-fold /4-fold volume), oxycodol (2.4- to 2.8-fold equiv) was added followed by NaH (8- to 12-fold equiv) and then PEGₙ-(OMs)₂. The reaction mixture was heated to 60-80°C and incubated with stirring until the reaction was confirmed by LC-MS analysis to be complete (depending on the chain length of PEG, 12-40 hours). The reaction was quenched with anhydrous methanol (10-fold volume). The reaction mixture was concentrated under reduced pressure to dryness. The residue was purified using a Biotage flash purification preparative liquid chromatograph to give a yellow to orange oil, yield: 15-30%. 6-O-PEGₙ-(oxycodol)₂ free alkali (n = 1, 2, 3, 4, 5, 6, 7, 8, or 9) were prepared by this method, and the products were confirmed by ¹H NMR and LC-MS.

### Step 3: Preparation of 6-0- PEGₙ-(oxycodol)₂ Hydrochloride

6-O-PEGₙ-(oxycodol)₂ free alkali was dissolved in dichloromethane (16-fold volume) and 2 M HCl in diethyl ether (12-fold volume) was added. The reaction mixture was stirred at room temperature for 2 hours and evaporated under reduced pressure to remove the solvent. The residue was dissolved in anhydrous methanol (16-fold volume) and filtered. The filtrate was concentrated under reduced pressure to dryness to give a yellow to orange oil, yield: 95-100%, HPLC purity (UV254): 95-98%. The compounds prepared by this method include: α-6-O-PEG₁-(oxycodol)₂; α-6-O-PEG₂-(oxycodol)₂; α-6-O-PEG₃-(oxycodol)₂; α-6-O-PEG₄-(oxycodol)₂; α-6-O-PEG₅-(oxycodol)₂; α-6-O-PEG₆-(oxycodol)₂; α-6-O-PEG₇-(oxycodol)₂; α-6-O-PEG₈-(oxycodol)₂ and α-6-O-PEG₉-(oxycodol)₂. The products were confirmed by ¹H NMR and LC-MS.

### Example 5: Preparation of PEGₙ-(fentanyl)₂ Conjugate

The first scheme was to covalently bond a PEG oligomer to the phenyl group of phenylethylamine, and the specific preparation method was as follows:

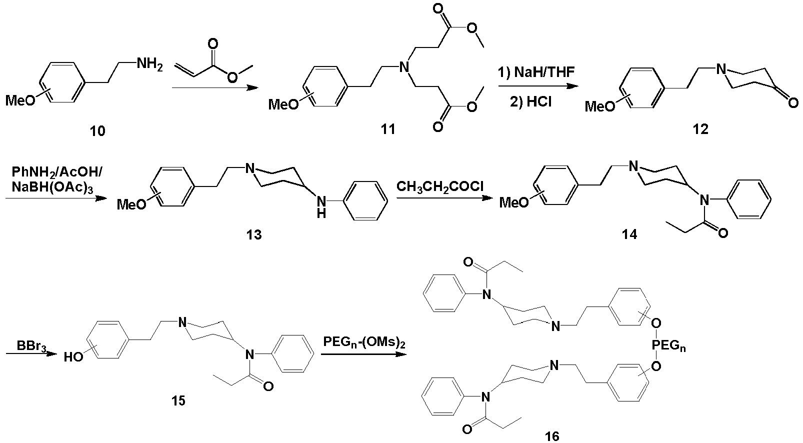

### Step 1: Preparation of N,N-bis(3-methoxycarbonylpropyl)-2-(methoxy-substituted phenyl)ethylamine (11)

To a reaction flask was added methyl acrylate (5.16 g, 60 mmol), 2-(methoxy-substituted phenyl)ethylamine (25 mmol) and an aqueous solution (25 mL) in which boric acid (0.15 g, 2.5 mmol) was dissolved. The mixture was stirred at room temperature for 7 h. The organic layer was separated, dried over anhydrous sodium sulfate, and evaporated under reduced pressure to remove the excess methyl acrylate to give a pale yellow oil, yield: 80-95%. The product was confirmed by ¹H NMR.

### Step 2: Preparation of N-[2-(methoxy-substituted phenyl)ethyl]piperidone (12)

To a reaction flask, sodium hydride (5.20 g, 60%, 130 mmol) and anhydrous tetrahydrofuran (72 mL) were added and heated to reflux. A solution of Intermediate **11** (50 mmol) in anhydrous tetrahydrofuran (18 mL) was added dropwise, and the dropping speed was controlled so that the bubbles smoothly overflowed. After the addition was complete, the mixture was continuously stirred and refluxed for 2 h, cooled to room temperature, and evaporated under reduced pressure to remove the solvent. Concentrated hydrochloric acid (60 mL) was slowly added to the reaction flask, and a large amount of a yellow solid precipitated. The mixture was heated and stirred to completely dissolve the solid and heating was continued until reflux reaction for 1.5 h. After cooling to room temperature, the pH of the reaction solution was adjusted to 9 to 10 with sodium carbonate solution, and a large amount of a pale yellow solid precipitated. The mixture was filtered to give a yellow crude product which was recrystallized from n-hexane to give a bright yellow solid, yield: 74-87%. The product was confirmed by ¹H NMR.

### Step 3: Preparation of 4-anilino-N-[2-(methoxy-substituted phenyl)ethyl]piperidine (13)

In an ice-water bath, aniline (8.24 g, 88.5 mmol) was dissolved in dichloromethane (240 mL) with stirring. Glacial acetic acid (5.0 mL, 88.5 mmol) was added dropwise. A solution of Intermediate **12** (88.5 mmol) in dichloromethane (60 mL) was added to the mixture followed by the slow addition of sodium triacetoxyborohydride (28.1 g, 132.8 mmol). The reaction mixture was stirred at room temperature for 14 h. Then methanol (100 mL) was added and finally saturated NaHCO₃ (300 mL) was added for liquid separation. The organic phase was washed with saturated brine (3×100 mL), dried over anhydrous sodium sulfate and concentrated under reduced pressure to give a pale brown solid, which was purified using a flash purification preparative liquid chromatograph to give a pale yellow solid, yield: 85-93%. The product was confirmed by ¹H NMR.

### Step 4: Preparation of N-{1-[2-(methoxy-substituted phenyl)ethyl]-4-piperidinyl}-N-phenylpropanamide (14)

Intermediate **13** (4.8 mmol) was dissolved in dichloromethane (40 mL). Diisopropylethylamine (1.68 mL, 9.6 mmol) was added. The solution was cooled in an ice-water bath. Propionyl chloride (0.83 mL, 9.6 mmol) was added dropwise. The reaction mixture was stirred at room temperature for 2 h. Then distilled water (40 mL) was added for liquid separation. The organic phase was washed sequentially with saturated brine (50 mL) and saturated NaHCO₃ (50 mL), dried over anhydrous sodium sulfate, and concentrated under reduced pressure to give a yellow solid, which was purified using a flash purification preparative liquid chromatograph to give a pale yellow solid, yield: 90-96%. The product was confirmed by ¹H NMR.

### Step 5: Preparation of N- {1-[2-(hydroxy-substituted phenyl)ethyl]-4-piperidinyl}-N-phenylpropanamide (15)

To a solution of Intermediate **14** (4.9 mmol) in dichloromethane (50 mL) was added boron tribromide (2.45 g, 9.8 mmol) at -78°C. The mixture was stirred for 2 h. Distilled water (25 mL) was added for liquid separation. The aqueous layer was extracted with dichloromethane (2×25 mL). The combined organic layer was washed with saturated brine (50 mL), dried over anhydrous sodium sulfate, and filtered. The filtrate was concentrated under reduced pressure. The residue was purified using a flash purification preparative liquid chromatograph to give a pale yellow solid, yield: 87-94%. The product was confirmed by ¹H NMR.

### Step 6: Preparation of PEGₙ-(fentanyl)₂-A Conjugate (16)

Intermediate **15** (2.7 mmol) and PEGₙ-(OMs)₂ (0.9 mmol) were dissolved in anhydrous acetonitrile (40 mL). Anhydrous K₂CO₃ (0.5 g, 3.6 mmol) was added. The mixture was heated to reflux and stirred and reacted for 16 h. The mixture was evaporated under reduced pressure to remove the solvent. The residue was dissolved in dichloromethane (100 mL). The organic layer was washed with distilled water (3×50 mL), dried over anhydrous sodium sulfate, and concentrated under reduced pressure. The residue was purified using a flash purification preparative liquid chromatograph to give a pale yellow solid, yield: 75-83%. The compounds prepared by the first scheme include: meta-substitutions PEG₁-(fentanyl)₂-Am, PEG₂-(fentanyl)₂-Am, PEG₃-(fentanyl)₂-Am, PEG₄-(fentanyl)₂-Am, PEG₅-(fentanyl)₂-Am, PEG₆-(fentanyl)₂-Am, PEG₇-(fentanyl)₂-Am, PEG₈-(fentanyl)₂-Am, and PEG₉-(fentanyl)₂-Am; para-substitutions PEG₁-(fentanyl)₂-Ap, PEG₂-(fentanyl)₂-Ap, PEG₃-(fentanyl)₂-Ap, PEG₄-(fentanyl)₂-Ap, PEG₅-(fentanyl)₂-Ap, PEG₆-(fentanyl)₂-Ap, PEG₇-(fentanyl)₂-Ap, PEG₈-(fentanyl)₂-Ap, and PEG₉-(fentanyl)₂-Ap; and ortho-substitutions PEG₁-(fentanyl)₂-Ao, PEG₂-(fentanyl)₂-Ao, PEG₃-(fentanyl)₂-Ao, PEG₄-(fentanyl)₂-Ao, PEG₅-(fentanyl)₂-Ao, PEG₆-(fentanyl)₂-Ao, PEG₇-(fentanyl)₂-Ao, PEG₈-(fentanyl)₂-Ao, and PEG₉-(fentanyl)₂-Ao. The products were confirmed by ¹H NMR and LC-MS.

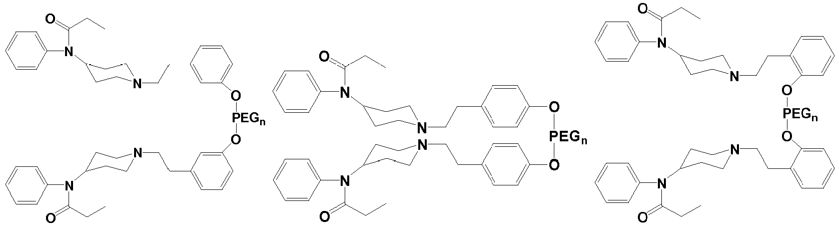

The second scheme was to covalently bond a PEG oligomer to the phenyl group of aniline, and the specific preparation method was as follows:

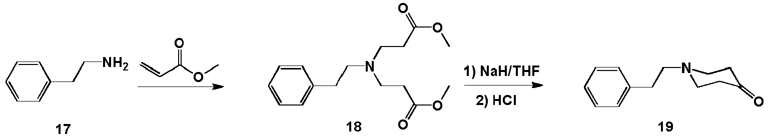

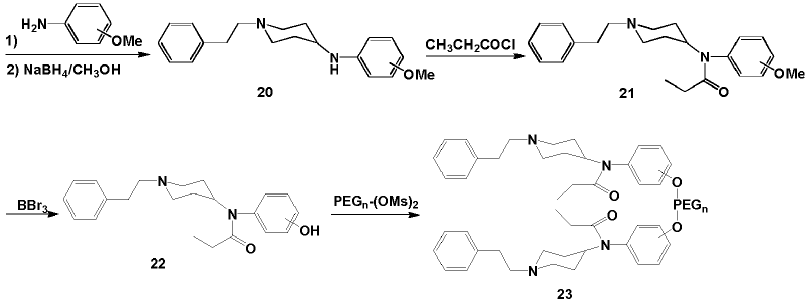

### Step 1: Preparation of N,N-bis(3-methoxycarbonylpropyl)-2-phenylethylamine (18)

To a reaction flask was added methyl acrylate (5.16 g, 60 mmol), phenethylamine (3.03 g, 25 mmol) and an aqueous solution (25 mL) in which boric acid (0.15 g, 2.5 mmol) was dissolved. The mixture was stirred at room temperature for 7 h. The organic layer was separated, dried over anhydrous sodium sulfate, and evaporated under reduced pressure to remove the excess methyl acrylate to give 6.7 g of a pale yellow oil, yield: 92%. ¹H NMR (400 MHz, CDCl₃) δ 7.31 (t, 2H), 7.22 (d, 2H), 7.08 (t, 1H), 3.67 (s, 6H), 2.75 (t, 4H), 2.69 (t, 2H), 2.65(t, 2H), 2.35(t, 4H).

### Step 2: Preparation of N-(2-phenylethyl)piperidone (19)

To a reaction flask, sodium hydride (5.20 g, 60%, 130 mmol) and anhydrous tetrahydrofuran (72 mL) were added and heated to reflux. A solution of Intermediate **18** (14.7 g, 50 mmol) in anhydrous tetrahydrofuran (18 mL) was added dropwise, and the dropping speed was controlled so that the bubbles smoothly overflowed. After the addition was complete, the mixture was continuously stirred and refluxed for 2 h, cooled to room temperature, and evaporated under reduced pressure to remove the solvent. Concentrated hydrochloric acid (60 mL) was slowly added to the reaction flask, and a large amount of a yellow solid precipitated. The mixture was heated and stirred to completely dissolve the solid and heating was continued until reflux reaction for 1.5 h. After cooling to room temperature, the pH of the reaction solution was adjusted to 9 to 10 with sodium carbonate solution, and a large amount of a pale yellow solid precipitated. The mixture was filtered to give a yellow crude product which was recrystallized from n-hexane to give 8.6 g of a bright yellow solid, yield: 85%. ¹H NMR (400 MHz, CDCl₃) δ 7.32 (m, 2H), 7.21 (m, 3H), 2.83 (m, 2H), 2.72 (t, 4H), 2.74 (m, 2H), 2.47 (t, 4H).

### Step 3: Preparation of 4-(methoxy-substituted anilino)-N-(2-phenylethyl)piperidine (20)

In an ice-water bath, methoxy-substituted aniline (88.5 mmol) was dissolved in dichloromethane (240 mL) with stirring. Glacial acetic acid (5.0 mL, 88.5 mmol) was added dropwise. A solution of Intermediate **19** (18.0 g, 88.5 mmol) in dichloromethane (60 mL) was added to the mixture followed by the slow addition of sodium triacetoxyborohydride (28.1 g, 132.8 mmol). The reaction mixture was stirred at room temperature for 14 h. Then methanol (100 mL) was added and finally saturated NaHCO₃ (300 mL) was added for liquid separation. The organic phase was washed with saturated brine (3×100 mL), dried over anhydrous sodium sulfate and concentrated under reduced pressure to give a pale brown solid, which was purified using a flash purification preparative liquid chromatograph to give a pale yellow solid, yield: 85-93%. The product was confirmed by ¹H NMR.

### Step 4: Preparation of N-[1-(2-phenylethyl)-4-piperidinyl]-N-(methoxy-substituted phenyl)propanamide (21)

Intermediate **20** (4.8 mmol) was dissolved in dichloromethane (40 mL). Diisopropylethylamine (1.68 mL, 9.6 mmol) was added. The solution was cooled in an ice-water bath. Propionyl chloride (0.83 mL, 9.6 mmol) was added dropwise. The reaction mixture was stirred at room temperature for 2 h. Then distilled water (40 mL) was added for liquid separation. The organic phase was washed sequentially with saturated brine (50 mL) and saturated NaHCO₃ (50 mL), dried over anhydrous sodium sulfate, and concentrated under reduced pressure to give a yellow solid, which was purified using a flash purification preparative liquid chromatograph to give a pale yellow solid, yield: 90-96%. The product was confirmed by ¹H NMR.

### Step 5: Preparation of N-[1-(2-phenylethyl)-4-piperidinyl]-N-(hydroxy-substituted phenyl)propanamide (22)

To a solution of Intermediate **21** (4.9 mmol) in dichloromethane (50 mL) was added boron tribromide (2.45 g, 9.8 mmol) at -78°C. The mixture was stirred for 2 h. Distilled water (25 mL) was added for liquid separation. The aqueous layer was extracted with dichloromethane (2×25 mL). The combined organic layer was washed with saturated brine (50 mL), dried over anhydrous sodium sulfate, and filtered. The filtrate was concentrated under reduced pressure. The residue was purified using a flash purification preparative liquid chromatograph to give a pale yellow solid, yield: 87-94%. The product was confirmed by ¹H NMR.

### Step 6: Preparation of PEGₙ-(fentanyl)₂-B Conjugate (23)

Intermediate **22** (2.7 mmol) and PEGₙ-(OMs)₂ (0.9 mmol) were dissolved in anhydrous acetonitrile (40 mL). Anhydrous K₂CO₃ (0.5 g, 3.6 mmol) was added. The mixture was heated to reflux and stirred and reacted for 16 h. The mixture was evaporated under reduced pressure to remove the solvent. The residue was dissolved in dichloromethane (100 mL). The organic layer was washed with distilled water (3×50 mL), dried over anhydrous sodium sulfate and concentrated under reduced pressure. The residue was purified using a flash purification preparative liquid chromatograph to give a pale yellow solid, yield: 75-83%. The compounds prepared by the second scheme include: meta-substitutions PEG₁-(fentanyl)₂-Bm, PEG₂-(fentanyl)₂-Bm, PEG₃-(fentanyl)₂-Bm, PEG₄-(fentanyl)₂-Bm, PEG₅-(fentanyl)₂-Bm, PEG₆-(fentanyl)₂-Bm, PEG₇-(fentanyl)₂-Bm, PEG₈-(fentanyl)₂-Bm, and PEG₉-(fentanyl)₂-Bm; para-substitutions PEG₁-(fentanyl)₂-Bp, PEG₂-(fentanyl)₂-Bp, PEG₃-(fentanyl)₂-Bp, PEG₄-(fentanyl)₂-Bp, PEG₅-(fentanyl)₂-Bp, PEG₆-(fentanyl)₂-Bp, PEG₇-(fentanyl)₂-Bp, PEG₈-(fentanyl)₂-Bp, and PEG₉-(fentanyl)₂-Bp; and ortho-substitutions PEG₁-(fentanyl)₂-Bo, PEG₂-(fentanyl)₂-Bo, PEG₃-(fentanyl)₂-Bo, PEG₄-(fentanyl)₂-Bo, PEG₅-(fentanyl)₂-Bo, PEG₆-(fentanyl)₂-Bo, PEG₇-(fentanyl)₂-Bo, PEG₈-(fentanyl)₂-Bo, and PEG₉-(fentanyl)₂-Bo. The products were confirmed by ¹H NMR and LC-MS.

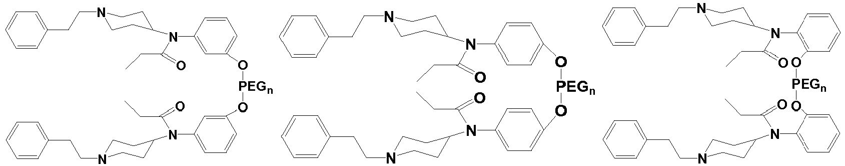

### Example 6: Preparation of PEGₙ-(OMs)₃

To a 250 mL three-neck flask was added a substrate PEG₁-(OH)₃ (10.0 g, 42 mmol) and dichloromethane (100 mL). The mixture was cooled to 0°C with stirring. Triethylamine (13.9 g, 168 mmol) was added dropwise. The reaction mixture was stirred for 10 minutes. Methanesulfonyl chloride (19.2 g, 168 mmol) was added to the reaction mixture over 5-10 minutes. The mixture was stirred at room temperature for 12 hours. The progress of the reaction was monitored by TLC. After the reaction was complete, distilled water was added to the reaction mixture, followed by extraction with dichloromethane (3×100 mL). The organic layers were combined, washed with distilled water (3×100 mL), dried over anhydrous sodium sulfate and concentrated under reduced pressure to dryness using a rotary evaporator to give 16.3 g of a brown oil, yield: 87.9%. ¹H NMR (400 MHz, CDCl₃): δ 4.46 (t, 6H), 4.07 (t, 6H), 3.92 (t, 6H), 3.76 (t, 9H), 2.69 (s, 6H).

Other PEGₙ-(OMs)₃ (n = 2, 3, 4, 5, 6, or 7) were prepared with the same method and their structure were confirmed by mass spectrometry and ₁H NMR spectroscopy.

### Example 7: Preparation of PEGₙ-(morphine)₃ Conjugate

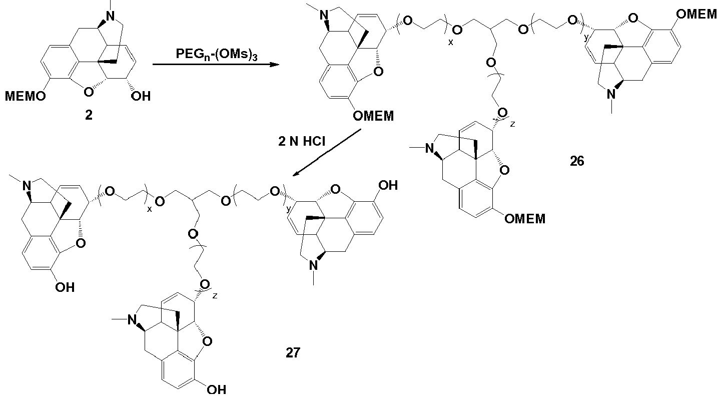

### Step 1: Preparation of 6-O-PEGₙ-(3-O-MEM-morphine)₃ (26)

To a mixed solution of toluene/DMF (8-fold/4-fold volume) was added 3-O-MEM-morphine (3.6- to 4.5-fold equiv) followed by NaH (12- to 18-fold equiv) and then PEGₙ-(OMs)₃. The reaction mixture was heated to 55-75°C and incubated with stirring until the reaction was confirmed by LC-MS analysis to be complete (depending on the chain length of PEG, 12-40 hours). The reaction was quenched with anhydrous methanol (10-fold volume). The reaction mixture was concentrated to dryness under reduced pressure. The residue was purified using a Biotage flash purification preparative liquid chromatograph to give a yellow to orange oil, yield: 15-30%. 6-O-PEGₙ-(3-O-MEM-morphine)₃ (n = 1, 2, 3, 4, 5, 6, or 7) were prepared by this method, and the products were confirmed by ¹H NMR and LC-MS.

### Step 2: Preparation of 6-O-PEGₙ-(morphine)₃ Hydrochloride (27)

6-O-PEGₙ-(3-O-MEM-morphine)₃ was dissolved in dichloromethane (20-fold volume) and 2 M HCl in diethyl ether (18-fold volume) was added. The reaction mixture was stirred at room temperature for 2 hours and evaporated under reduced pressure to remove the solvent. The residue was dissolved in anhydrous methanol (20-fold volume) and filtered. The filtrate was concentrated under reduced pressure to dryness to give a yellow to orange oil, yield: 95-100%, HPLC purity (UV254): 95-98%. The compounds prepared by this method include: α-6-O-PEG₁-(morphine)₃; α-6-O-PEG₂-(morphine)₃; α-6-O-PEG₃-(morphine)₃; α-6-O-PEG₄-(morphine)₃; α-6-O-PEG₅-(morphine)₃; α-6-O-PEG₆-(morphine)₃; and α-6-O-PEG₇-(morphine)₃. The products were confirmed by ¹H NMR and LC-MS.

### Example 8: Preparation of PEGₙ-(codeine)₃ Conjugate

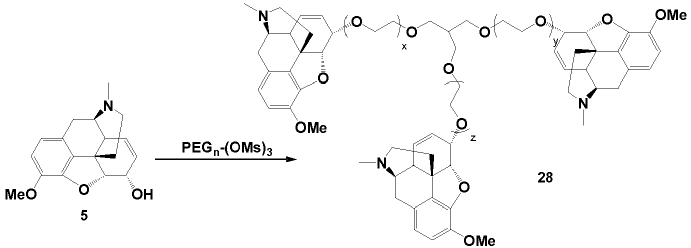

### Step 1: Preparation of 6-O-PEGₙ-(codeine)₃ Free Alkali (28)

To a mixed solution of toluene/DMF (24-fold/1-fold volume) was added codeine (3.6- to 4.5-fold equiv) followed by NaH (12- to 18-fold equiv) and then PEGₙ-(OMs)₃. The reaction mixture was heated to 45-65°C and incubated with stirring until the reaction was confirmed by LC-MS analysis to be complete (depending on the chain length of PEG, 12-48 hours). The reaction was quenched with anhydrous methanol (10-fold volume). The reaction mixture was concentrated under reduced pressure to dryness. The residue was purified using a Biotage flash purification preparative liquid chromatograph to give a yellow to orange oil, yield: 12-25%. 6-O-PEGₙ-(codeine)₃ free alkali (n=l, 2, 3, 4, 5, 6, or 7) were prepared by this method, and the products were confirmed by ¹H NMR and LC-MS.

### Step 2: Preparation of 6-O-PEGₙ-(codeine)₃ Hydrochloride

6-O-PEGₙ-(codeine)₃ free alkali was dissolved in dichloromethane (16-fold volume) and 2 M HCl in diethyl ether (12-fold volume) was added. The reaction mixture was stirred at room temperature for 2 hours and evaporated under reduced pressure to remove the solvent. The residue was dissolved in anhydrous methanol (16-fold volume) and filtered. The filtrate was concentrated under reduced pressure to dryness to give a yellow to orange oil, yield: 95-100%, HPLC purity (UV254): 95-98%. The compounds prepared by this method include: α-6-O-PEG₁-(codeine)₃; α-6-O-PEG₂-(codeine)₃; α-6-O-PEG₃-(codeine)₃; α-6-O-PEG₄-(codeine)₃; α-6-O-PEG₅-(codeine)₃; α-6-O-PEG₆-(codeine)₃; and α-6-O-PEG₇-(codeine)₃. The products were confirmed by ¹H NMR and LC-MS.

### Example 9: Preparation of PEGₙ-(oxycodol)₃ Conjugate

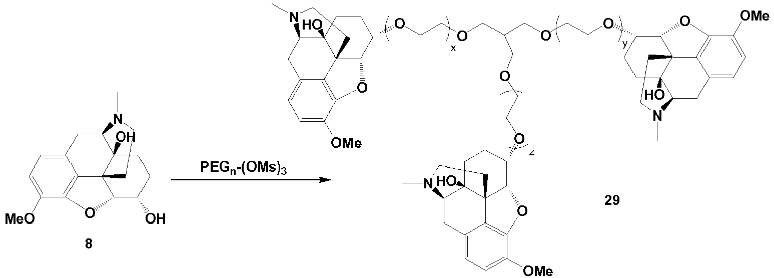

### Step 1: Preparation of 6-O-PEGₙ-(oxycodol)₃ Free Alkali (29)

To a mixed solution of toluene/DMF (8-fold/4-fold volume), oxycodol (3.6- to 4.5-fold equiv) was added followed by NaH (12- to 18-fold equiv) and then PEGₙ-(OMs)₃. The reaction mixture was heated to 60-80°C and incubated with stirring until the reaction was confirmed by LC-MS analysis to be complete (depending on the chain length of PEG, 12-40 hours). The reaction was quenched with anhydrous methanol (10-fold volume). The reaction mixture was concentrated under reduced pressure to dryness. The residue was purified using a Biotage flash purification preparative liquid chromatograph to give a yellow to orange oil, yield: 15-30%. 6-O-PEGₙ-(oxycodol)₃ free alkali (n = 1, 2, 3, 4, 5, 6, or 7) were prepared by this method, and the products were confirmed by ¹H NMR and LC-MS.

### Step 2: Preparation of 6-O-PEGₙ-(oxycodol)₃ Hydrochloride

6-O-PEGₙ-(oxycodol)₃ free alkali was dissolved in dichloromethane (20-fold volume) and 2 M HCl in diethyl ether (18-fold volume) was added. The reaction mixture was stirred at room temperature for 2 hours and evaporated under reduced pressure to remove the solvent. The residue was dissolved in anhydrous methanol (20-fold volume) and filtered. The filtrate was concentrated under reduced pressure to dryness to give a yellow to orange oil, yield: 95-100%, HPLC purity (UV254): 95-98%. The compounds prepared by this method include: α-6-O-PEG₁-(oxycodol)₃; α-6-O-PEG₂-(oxycodol)₃; α-6-O-PEG₃-(oxycodol)₃; α-6-O-PEG₄-(oxycodol)₃; α-6-O-PEG₅-(oxycodol)₃; α-6-O-PEG₆-(oxycodol)₃; α-6-O-PEG₇-(oxycodol)₃. The products were confirmed by ¹H NMR and LC-MS.

### Example 10: Preparation of PEGₙ-(fentanyl)₃ Conjugate

The first scheme was to covalently bond a PEG oligomer to the phenyl group of phenylethylamine, and the specific preparation method of PEGₙ-(fentanyl)₃-A conjugate **(30)** was as follows:

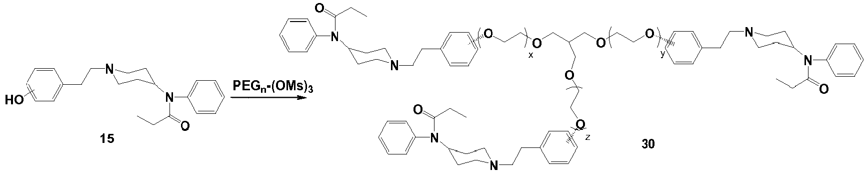

Intermediate **15** (2.7 mmol) and PEGₙ-(OMs)₃ (0.9 mmol) were dissolved in anhydrous acetonitrile (40 mL). Anhydrous K₂CO₃ (0.75 g, 5.4 mmol) was added. The mixture was heated to reflux and stirred and reacted for 16 h. The mixture was evaporated under reduced pressure to remove the solvent. The residue was dissolved in dichloromethane (100 mL). The organic layer was washed with distilled water (3×50 mL), dried over anhydrous sodium sulfate and concentrated under reduced pressure. The residue was purified using a flash purification preparative liquid chromatograph to give a pale yellow solid, yield: 75-83%. PEGₙ-(fentanyl)₃-A conjugates (n=l, 2, 3, 4, 5, 6, or 7) were prepared by the method of the first scheme, and the products were confirmed by ¹H NMR and LC-MS.

The second scheme was to covalently bond a PEG oligomer to the phenyl group of aniline, and the specific preparation method of PEGₙ-(fentanyl)₃-B conjugate **(31)** was as follows:

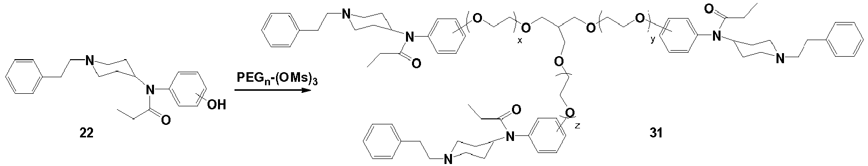

Intermediate **22** (2.7 mmol) and PEGₙ-(OMs)₃ (0.9 mmol) were dissolved in anhydrous acetonitrile (40 mL). Anhydrous K₂CO₃ (0.75 g, 5.4 mmol) was added. The mixture was heated to reflux and stirred and reacted for 16 h. The mixture was evaporated under reduced pressure to remove the solvent. The residue was dissolved in dichloromethane (100 mL). The organic layer was washed with distilled water (3×50 mL), dried over anhydrous sodium sulfate and concentrated under reduced pressure. The residue was purified using a flash purification preparative liquid chromatograph to give a pale yellow solid, yield: 75-83%. PEGₙ-(fentanyl)₃-B conjugates (n=l, 2, 3, 4, 5, 6, or 7) were prepared by the method of the second scheme, and the products were confirmed by ¹H NMR and LC-MS.

### Example 11: Preparation of PEGₙ-(OMs)₄

To a 250 mL three-necked flask was added a substrate PEG₁-(OH)₄ (10.0 g, 32 mmol) and dichloromethane (100 mL). The mixture was stirred and cooled to 0°C. Triethylamine (15.5 g, 153.6 mmol) was added dropwise. The reaction mixture was stirred for 10 minutes. Methanesulfonyl chloride (22.2 g, 153.6 mmol) was added to the reaction mixture over 5-10 minutes. The mixture was stirred at room temperature for 12 hours. The progress of the reaction was monitored by TLC. After the reaction was complete, distilled water was added to the reaction mixture, followed by extraction with dichloromethane (3×100 mL). The organic layers were combined, washed with distilled water (3×100 mL), dried over anhydrous sodium sulfate and concentrated under reduced pressure to dryness using a rotary evaporator to give 17.6 g of a brown oil, yield: 87.3%. ¹H NMR (400 MHz, CDCl₃): δ 4.48 (t, 8H), 4.42 (t, 8H), 4.17 (t, 8H), 3.78 (t, 8H).

Other PEGₙ-(OMs)₄ (n=2, 3, 4, or 5) were prepared in the same manner, and their structure were confirmed by mass spectrometry and ¹H NMR spectroscopy.

### Example 12: Preparation of PEGₙ-(morphine)₄ Conjugate

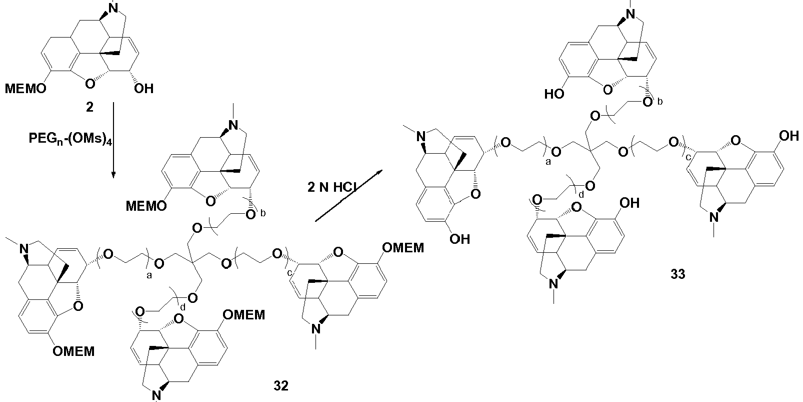

### Step 1: Preparation of 6-O-PEGₙ-(3-O-MEM-morphine)₄ (32)

To a mixed solution of toluene/DMF (8-fold/4-fold volume) was added 3-O-MEM-morphine (4.8- to 6-fold equiv) followed by NaH (16- to 24-fold equiv) and then PEGₙ-(OMs)₄. The reaction mixture was heated to 55-75°C and incubated with stirring until the reaction was confirmed by LC-MS analysis to be complete (depending on the chain length of PEG, 12-40 hours). The reaction was quenched with anhydrous methanol (10-fold volume). The reaction mixture was concentrated under reduced pressure to dryness. The residue was purified using a Biotage flash purification preparative liquid chromatograph to give a yellow to orange oil, yield: 15-30%. 6-O-PEGₙ-(3-O-MEM-morphine)₄ (n = 1, 2, 3, 4, or 5) were prepared by this method, and the products were confirmed by ¹H NMR and LC-MS.

### Step 2: Preparation of 6-O-PEGₙ-(morphine)₄ Hydrochloride

6-O-PEGₙ-(3-O-MEM-morphine)₄ was dissolved in dichloromethane (20-fold volume) and 2 M HCl in diethyl ether (16-fold volume) was added. The reaction mixture was stirred at room temperature for 2 hours and evaporated under reduced pressure to remove the solvent. The residue was dissolved in anhydrous methanol (20-fold volume) and filtered. The filtrate was concentrated under reduced pressure to dryness to give a yellow to orange oil, yield: 95-100%, HPLC purity (UV254): 95-98%. The compounds prepared by this method include: α-6-O-PEG₁-(morphine)₄; α-6-O-PEG₂-(morphine)₄; α-6-O-PEG₃-(morphine)₄; α-6-O-PEG₄-(morphine)₄; and α-6-O-PEG₅-(morphine)₄. The products were confirmed by ¹H NMR and LC-MS.

### Example 13: Preparation of PEGₙ-(codeine)₄ Conjugate

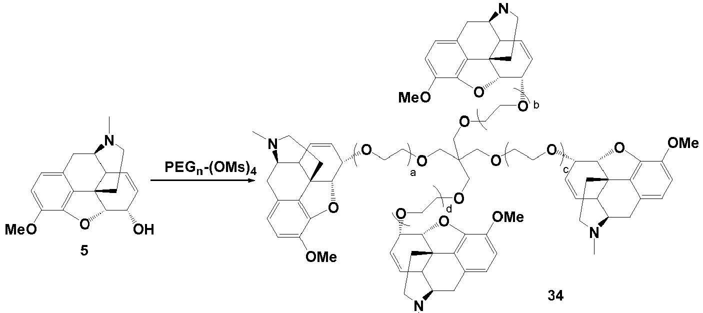

### Step 1: Preparation of 6-O-PEGₙ-(codeine)₄ Free Alkali (34)

To a mixed solution of toluene/DMF (24-fold/1-fold volume) was added codeine (4.8- to 6-fold equiv) followed by NaH (16- to 24-fold equiv) followed by PEGₙ-(OMs)₄. The reaction mixture was heated to 45-65°C and incubated with stirring until the reaction wasconfirmed by LC-MS analysis to be complete (depending on the chain length of PEG, 12-48 hours). The reaction was quenched with anhydrous methanol (10-fold volume). The reaction mixture was concentrated under reduced pressure to dryness. The residue was purified using a Biotage flash purification preparative liquid chromatograph to give a yellow to orange oil, yield: 12-25%. 6-O-PEGₙ-(codeine)₄ free alkali (n=l, 2, 3, 4, or 5) were prepared by this method, and the products were confirmed by ¹H NMR and LC-MS.

### Step 2: Preparation of 6-O-PEGₙ-(codeine)₄ Hydrochloride

6-O-PEGₙ-(codeine)₄ free alkali was dissolved in dichloromethane (20-fold volume) and 2 M HCl in diethyl ether (16-fold volume) was added. The reaction mixture was stirred at room temperature for 2 hours and evaporated under reduced pressure to remove the solvent. The residue was dissolved in anhydrous methanol (20-fold volume) and filtered. The filtrate was concentrated under reduced pressure to dryness to give a yellow to orange oil, yield: 95-100%, HPLC purity (UV254): 95-98%. The compounds prepared by this method include: α-6-O-PEG₁-(codeine)₄; α-6-O-PEGₙ-(codeine)₄; α-6-O-PEG₃-(codeine)₄; α-6-O-PEG₄-(codeine)₄; and α-6-O-PEG₅-(codeine)₄. The products were confirmed by ¹H NMR and LC-MS.

### Example 14: Preparation of PEGₙ-(oxycodol)₄ Conjugate (35)

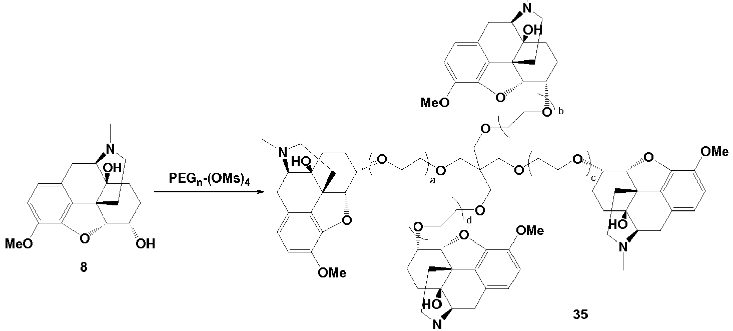

### Step 1: Preparation of 6-O-PEGₙ-(oxycodol)₄ Free Alkali (35)

To a mixed solution of toluene/DMF (8-fold/4-fold volume), oxycodol (4.8- to 6-fold equiv) was added followed by NaH (16- to 24-fold equiv) and then PEGₙ-(OMs)₄. The reaction mixture was heated to 60-80°C and incubated with stirring until the reaction was confirmed by LC-MS analysis to be complete (depending on the chain length of PEG, 12-40 hours). The reaction was quenched with anhydrous methanol (10-fold volume). The reaction mixture was concentrated under reduced pressure to dryness. The residue was purified using a Biotage flash purification preparative liquid chromatograph to give a yellow to orange oil, yield: 15-30%. 6-O-PEGₙ-(oxycodol)₄ free alkali (n = 1, 2, 3, 4, or 5) were prepared by this method, and the products were confirmed by ¹H NMR and LC-MS.

### Step 2: Preparation of 6-O-PEGₙ-(oxycodol)₄ Hydrochloride

6-O-PEGₙ-(oxycodol)₄ free alkali was dissolved in dichloromethane (20-fold volume) and 2 M HCl in diethyl ether (16-fold volume) was added. The reaction mixture was stirred at room temperature for 2 hours and evaporated under reduced pressure to remove the solvent. The residue was dissolved in anhydrous methanol (20-fold volume) and filtered. The filtrate was concentrated under reduced pressure to dryness to give a yellow to orange oil, yield: 95-100%, HPLC purity (UV254): 95-98%. The compounds prepared by this method include: α-6-O-PEG₁-(oxycodol)₄; α-6-O-PEG₂-(oxycodol)₄; α-6-O-PEG₃-(oxycodol)₄; α-6-O-PEG₄-(oxycodol)₄; and α-6-O-PEG₅-(oxycodol)₄. The products were confirmed by ¹H NMR and LC-MS.

### Example 15: Preparation of PEGₙ-(fentanyl)₄ Conjugate

The first scheme was to covalently bond a PEG oligomer to the phenyl group of phenylethylamine, and the specific preparation method of PEGₙ-(fentanyl)₄-A conjugate **(36)** was as follows:

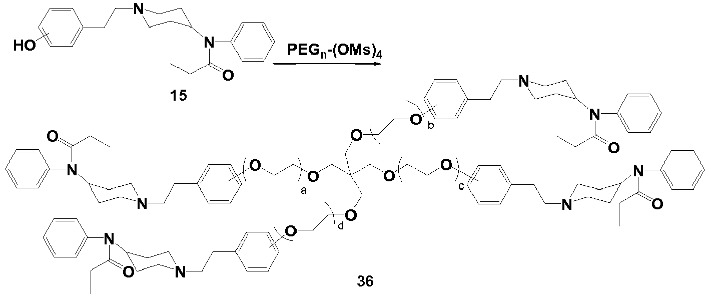

Intermediate **15** (3.6 mmol) and PEGₙ-(OMs)₄ (0.9 mmol) were dissolved in anhydrous acetonitrile (40 mL). Anhydrous K₂CO₃ (1.0 g, 7.2 mmol) was added. The mixture was heated to reflux and stirred and reacted for 16 h. The mixture was evaporated under reduced pressure to remove the solvent. The residue was dissolved in dichloromethane (100 mL). The organic layer was washed with distilled water (3×50 mL), dried over anhydrous sodium sulfate and concentrated under reduced pressure. The residue was purified using a flash purification preparative liquid chromatograph to give a pale yellow solid, yield: 75-83%. PEGₙ-(fentanyl)₄-A conjugates were prepared by the method of the first scheme, n was 1, 2, 3, 4, or 5, respectively, and the products were confirmed by ¹H NMR and LC-MS.

The second scheme was to covalently bond a PEG oligomer to the phenyl group of aniline, and the specific preparation method of PEGₙ-(fentanyl)₄-B conjugate **(37)** was as follows:

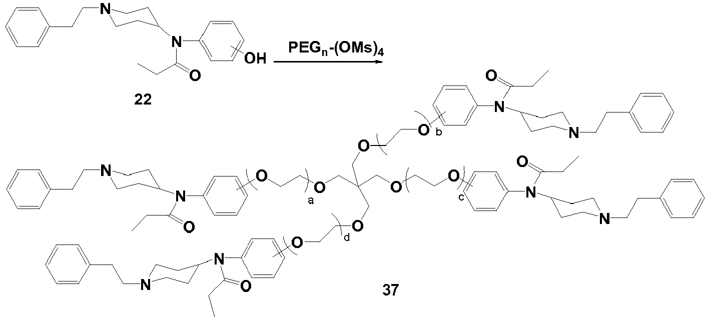

Intermediate **22** (3.6 mmol) and PEGₙ-(OMs)₄ (0.9 mmol) were dissolved in anhydrous acetonitrile (40 mL). Anhydrous K₂CO₃ (1.0 g, 7.2 mmol) was added. The mixture was heated to reflux and stirred for 16 h. The mixture was evaporated under reduced pressure to remove the solvent. The residue was dissolved in dichloromethane (100 mL). The organic layer was washed with distilled water (3×50 mL), dried over anhydrous sodium sulfate and concentrated under reduced pressure. The residue was purified using a flash purification preparative liquid chromatograph to give a pale yellow solid, yield: 75-83%. PEGₙ-(fentanyl)₄-B conjugates were prepared by the method of the second scheme, n was 1, 2, 3, 4, or 5, respectively, and the products were confirmed by ¹H NMR and LC-MS.

### Example 16: Pharmacokinetics Experiment

### 16.1 Test for Blood-Brain Barrier Permeability of the Pegylated Opioids

### Experimental method:

4-5 mL of cardiac blood (using heparin for anticoagulation) was collected from female SD rats after intravenous administration (at a dose of 5 mg/kg for each sample) at 10 min, 60 min and 180 min (3 animals per time point), respectively. The plasma was collected after centrifugation, and stored at 4°C until use. After blood collection, the SD rats were immediately sacrificed by decapitation. The whole brain tissue was collected, with the meninges and blood vessels peeled off, rinsed with a small amount of normal saline (NS), dried with a filter paper, and weighed. The brain tissue was added to normal saline 1.5 times the brain tissue weight, homogenized in an ice bath, placed in a test tube, capped, and stored at 4°C until analysis. The brain: plasma ratio for each sample was calculated based on the experimental results. The experimental results are shown in Table 1.

**Table 1**

| Test drugs | Plasma Concentration (ng/mL) | | | Brain Concentration (ng/g) | | | Brain: Plasma Ratio | | |
|---|---|---|---|---|---|---|---|---|---|
| | 10 min | 60 min | 180 min | 10 min | 60 min | 180 min | 10 min | 60 min | 180 min |
| Morphine | 1097 | 154 | 10 | 2698 | 223 | 35 | 2.46 | 1.45 | 3.50 |
| α-6-O-PEG₁-(morphine)₂ | 891 | 138 | 38 | 687 | 299 | 183 | 0.77 | 2.17 | 4.82 |
| α-6-O-PEG₂-(morphine)₂ | 1212 | 123 | 43 | 554 | 342 | 221 | 0.46 | 2.78 | 5.14 |
| α-6-O-PEG₃-(morphine)₂ | 1016 | 119 | 32 | 387 | 262 | 167 | 0.38 | 2.20 | 5.22 |
| α-6-O-PEG₄-(morphine)₂ | 1121 | 114 | 42 | 226 | 198 | 164 | 0.20 | 1.74 | 3.90 |
| α-6-O-PEG₅-(morphine)₂ | 926 | 101 | 51 | 125 | 123 | 95 | 0.13 | 1.22 | 1.86 |
| α-6-O-PEG₆-(morphine)₂ | 1204 | 123 | 72 | 163 | 133 | 111 | 0.14 | 1.08 | 1.54 |
| α-6-O-PEG₇-morphine) ₂ | 1171 | 117 | 71 | 177 | 111 | 105 | 0.15 | 0.95 | 1.48 |
| α-6-O-PEG₈-(morphine)₂ | 1233 | 167 | 90 | 140 | 153 | 127 | 0.11 | 0.92 | 1.41 |
| α-6-O-PEG₉-(morphine)₂ | 1234 | 201 | 101 | 152 | 125 | 123 | 0.12 | 0.62 | 1.22 |
| PEG₄-(fentanyl)₂-Am | 2316 | 134 | 83 | 189 | 179 | 133 | 0.08 | 1.34 | 1.60 |
| PEG₆-(fentanyl)₂-Am | 2212 | 435 | 108 | 157 | 161 | 144 | 0.07 | 0.37 | 1.33 |
| α-6-O-PEG₄-(codeine)₂ | 1657 | 233 | 43 | 121 | 110 | 107 | 0.07 | 0.47 | 2.49 |
| α-6-O-PEG₆-(codeine)₂ | 1745 | 445 | 67 | 102 | 100 | 97 | 0.06 | 0.22 | 1.45 |
| α-6-O-PEG₄-(oxycodol)₂ | 2280 | 92 | 11 | 143 | 136 | 123 | 0.06 | 1.48 | 11.18 |
| α-6-O-PEG₆-(oxycodol)₂ | 2012 | 504 | 38 | 132 | 118 | 112 | 0.07 | 0.23 | 2.95 |

The experimental results showed that after pegylation, the amount of morphine, fentanyl and other opioids entering the brain tissue decreased to some extent. Moreover, due to the increase in the molecular weight, their rate of elimination from the brain also correspondingly slowed down. At the three time points measured, the concentration of drugs in the brain fluctuated gently. At 180 minutes after administration, the concentrations of the pegylated drugs in the brain were all higher than that in blood. It is now widely accepted that the formation of addiction to opioids is associated with their rapid entry into the central nervous system. Therefore, the characteristics of the permeability through blood-brain barrier of the pegylated opioids are beneficial for opioids to exert long-lasting analgesic effects and reduce the risk of drug addiction. 16.2 Evaluation of Oral Bioavailability of the Pegylated Opioids

### Experimental method:

SD rats were anesthetized with an intraperitoneal injection of 1% pentobarbital sodium (40 mg/kg). The skin at the back and front of the neck was shaved and disinfected with iodophor. The skin at the right to the middle of the neck was cut open to expose the jugular vein. A venous catheter was inserted into the blood vessel and then ligated, and the skin at the opening was sutured. After the operation was completed, approximately 0.2 mL of heparin sodium solution and 0.1 mL of blocking solution were intraductally injected into the catheter, and thereafter replaced daily for a week. After a week, the rats with all the surgical wounds healed, the catheter fixed accurately and repeated blood collection unobstructed were used in the pharmacokinetic study of this project. The rats with successful surgery were randomly divided into groups with 3 in each group and fasted for 12 hours before the experiment. Each test drug was administered by both oral and intravenous routes at doses of 50 mg/kg and 10 mg/kg, respectively. Animals in the oral administration group were taken blood through the inserted catheter before and at 10 min, 30 min, 1 hr, 2 hr, 4 hr, 6 hr, 8 hr, and 24 hr after the administration; and those in the intravenous administration group were taken blood through the inserted catheter before and at 5 min, 10 min, 30 min, 1 hr, 2 hr, 4 hr, 8 hr and 24 hr after the administration. Blood samples were centrifuged to collect the plasma. The drug concentration of each sample was measured by LC-MS/MS. The experimental results are shown in Tables 2 to 3.

**Table 2: Pharmacokinetic parameters for intravenous administration of each test drug**

| Compounds | PK parameters | | | | | | |
|---|---|---|---|---|---|---|---|
| | CL | Vss | T_{1/2} | AUCₗₐₛₜ | AUC_{INF} | MRT_{INF} | Vz |
| | mL/min/ kg | L/kg | hr | hr^{∗}ng/m L | hr^{∗}ng/mL | hr | L/kg |
| Morphine | 373 | 18.9 | 0.8 | 42.5 | 44.8 | 0.5 | 29 |
| α-6-O-PEG₁-(morphine)₂ | 342 | 47.3 | 2.59 | 83 | 98 | 2.46 | 72 |
| α-6-O-PEG₂-(morphine)₂ | 307 | 37.2 | 2.88 | 90 | 107 | 2.08 | 52.1 |
| α-6-O-PEG₃-(morphine)₂ | 296 | 27.8 | 2.31 | 104 | 112 | 2.18 | 43 |
| α-6-O-PEG₄-(morphine)₂ | 238 | 64.3 | 4.71 | 122 | 137 | 6.12 | 94.3 |
| α-6-O-PEG₅-(morphine)₂ | 261 | 55.2 | 3.71 | 109 | 128 | 3.52 | 84 |
| α-6-O-PEG₆-(morphine)₂ | 204 | 38.6 | 3.42 | 144 | 158 | 3.22 | 57.7 |
| α-6-O-PEG₇-(morphine)₂ | 191 | 57.4 | 5.98 | 156 | 183 | 5.68 | 87 |
| α-6-O-PEG₈-(morphine)₂ | 221 | 84.7 | 6.83 | 162 | 188 | 10.8 | 125.2 |
| α-6-O-PEG₉-(morphine)₂ | 166 | 60.1 | 8.33 | 221 | 259 | 7.91 | 92 |

**Table 3: Pharmacokinetic parameters for oral administration of each test drug**

| Compounds | PK parameters | | | | | |
|---|---|---|---|---|---|---|
| | Tmax | Cmax | T_{1/2} | AUCₗₐₛₜ | AUC_{INF} | F |
| | hr | L/kg | hr | hr^{∗}ng/mL | hr^{∗}ng/mL | % |
| Morphine | 1.0 | 23 | 2.8 | 36 | 43 | 3.1 |
| α-6-O-PEG₁-(morphine)₂ | 1.0 | 56 | 11 | 78 | 92 | 3.7 |
| α-6-O-PEG₂-(morphine)₂ | 2 | 18 | 4.0 | 27 | 35 | 4.8 |
| α-6-O-PEG₃-(morphine)₂ | 2 | 12 | 5.1 | 15 | 18 | 3.2 |
| α-6-O-PEG₄-(morphine)₂ | 3 | 47 | 6.4 | 70 | 81 | 17.1 |
| α-6-O-PEG₅-(morphine)₂ | 2.0 | 75 | 7.8 | 129 | 156 | 28.2 |
| α-6-O-PEG₆-(morphine)₂ | 3 | 54 | 4.8 | 73 | 84 | 21.2 |
| α-6-O-PEG₇-(morphine)₂ | 2.0 | 23 | 7.3 | 22 | 28 | 7.0 |
| α-6-O-PEG₈-(morphine)₂ | 3 | 22 | 5.2 | 22 | 31 | 7.6 |
| α-6-O-PEG₉-(morphine)₂ | 3.0 | 11 | 11.5 | 11 | 17 | 5.2 |

The above experimental results showed that the oral bioavailability of the pegylated drugs increased to some extent compared with that of the original drugs. However, the oral bioavailability of the compounds with different lengths of PEG varied greatly. In, general, the compounds of PEG₄₋₆ have better oral bioavailability, and other compounds with too long or too short PEG chains showed significant absorption loss. In addition, with the increase of PEG length, the compounds with various PEG forms exhibited a lower clearance rate, a higher distribution volume at steady state, and a longer elimination half-life.

### 16.3 Pharmacokinetic Experiment of Single-, Three-, and Four-Ended Compounds

In order to investigate the pharmacokinetic properties of the pegylated morphine with other modes of linkage, the brain permeability, oral bioavailability, and blood pharmacokinetic properties of the single-, three-, and four-ended compounds with 2-, 4-, 6-unit PEG were evaluated according to the above-described experimental method. The results showed that the three- and four-ended compounds has significantly decreased oral availability compared with the double-ended compounds, which ranged from 2.1% to 7.6% and 1.9% to 5.3%, respectively; and has the same tendency to brain permeability, which was significantly lower than that of the double-ended compounds, and was equivalent to 10%-30% of the double-ended compounds with the same unit of PEG. The single-ended compounds had significantly better oral availability and brain permeability than the double-ended compounds with the same unit of PEG, and both the indicators were approximately 1.5 times greater than those of the double-ended compounds.

### Example 17: Analgesic Experiment

### 17.1 Writhing Test

### Experimental Method:

ICR mice were randomly divided into groups with 10 in each group: test groups: the mice were administered intravenously with each test drug at a dose of 10 mg/kg, respectively, and a control group: the mice were given an equal amount of distilled water. Half an hour after the administration, mice were intraperitoneally injected with 0.6% glacial acetic acid (at 0.2 mL for each). The number of writhing responses (abdominal contraction into an "S" shape, body twist, hind limb extension, and creep, etc.) of mice within 20 min was recorded. The experimental results are shown in Table 4.

**Table 4**

| Test drugs | N | Dose (mg/kg) | Number of Writhing |
|---|---|---|---|
| NS | 10 | - | 42.5±7.2 |
| Morphine | 10 | 5 | 10.2±2.1 |
| Fentanyl | 10 | 0.075 | 8.8±1.2 |
| Codeine | 10 | 30 | 13.4±3.1 |
| Oxycodone | 10 | 5 | 9.9±3.2 |
| α-6-O-mPEG₄-morphine * | 10 | 10 | 12.1±2.3 |
| α-6-O-mPEG₆-morphine * | 10 | 10 | 15.3±3.4 |
| α-6-O-PEG₄-(morphine)₂ | 10 | 10 | 20.1±2.3 |
| α-6-O-PEG₆-(morphine)₂ | 10 | 10 | 24.3±3.4 |
| α-6-O-PEG₄-(morphine)₃ | 10 | 10 | 28.8±1.9 |
| α-6-O-PEG₆-(morphine)₃ | 10 | 10 | 34.2±2.2 |
| α-6-O-PEG₃-(morphine)₄ | 10 | 10 | 34.6±5.3 |
| α-6-O-PEG₅-(morphine)₄ | 10 | 10 | 32.3±6.3 |
| PEG₄-(fentanyl)₂-Am | 10 | 1 | 17.8±3.8 |
| PEG₆-(fentanyl)₂-Am | 10 | 1 | 19.7±1.8 |
| α-6-O-PEG₄-(codeine)₂ | 10 | 30 | 31.3±4.1 |
| α-6-O-PEG₆-(codeine)₂ | 10 | 30 | 34.1±3.3 |
| α-6-O-PEG₄-(oxycodol)₂ | 10 | 10 | 18.8±2.8 |
| α-6-O-PEG₆-(oxycodol)₂ | 10 | 10 | 20.1±2.1 |

| | | | |
|---|---|---|---|
| * Prepared according to the method of the literature (CN 102159249A). | | | |

The experimental results showed that the analgesic activity of all opioid analgesics decreased after pegylation was reduced. As a whole, the overall analgesic effect of the test drugs tended to decrease with the increase of PEG units. The single-ended and double-ended samples had obvious analgesic effects, and the three- and four-end samples had relatively poor analgesic effect.

### 17.2 Tail Flick Test

### Experimental Method:

ICR mice were randomized according to body weight with 10 in each group, and injected with each control drug and test drug by tail vein. The dose of morphine control drug was 5 mg/kg, and the dose of other test drugs were the same as the Writhing Test. The heat pain test was performed using a heat radiation pain tester, and the heat radiation value was adjusted to a constant 50 units. At the time of detection, the time began to count from when the mouse was quiet until the time when the mouse flicked their tails. The time displayed on the heat radiation pain tester was recorded in units of seconds. The heat pain values before (base value), and at 15 min, 1 hr, 2 hr, 6 hr and 24 hr after administration were measured. The experimental results are shown in Table 5.

**Table 5**

| Groups | Heat pain value (seconds) | | | | | |
|---|---|---|---|---|---|---|
| | 0 hr | 0.25 hr | 1 hr | 2 hr | 6 hr | 24 hr |
| NS | 14.7±3.7 | 14.0±4.2 | 13.9±1.8 | 14.4±1.9 | 13.8±1.6 | 14.4±1.6 |
| Morphine | 14.0±2.2 | 22.7±5.1 | 42.4±5.1 | 29.0±4.7 | 13.5±1.2 | 13.8±1.5 |
| α-6-O-mPEG₄-morphine | 13.7±2.7 | 19.7±2.5 | 34.8±2.4 | 32.2±4.7 | 18.2±5.8 | 13.6±2.1 |
| α-6-O-mPEG₆-morphine | 13.5±2.6 | 15.7±4.7 | 32.9±3.9 | 37.0±4.3 | 19.9±3.7 | 14.7±2.7 |
| α-6-O-PEG₄-(morphine)₂ | 13.2±1.6 | 18.7±3.2 | 31.7±9.6 | 35.2±5.3 | 36.6±3.4 | 19.7±3.4 |
| α-6-O-PEG₆-(morphine)₂ | 13.3±3.2 | 15.8±5.8 | 34.2±6.8 | 36.8±2.9 | 35.7±4.6 | 21.9±4.3 |
| PEG₄-(fentanyl)₂-Am | 13.5±2.7 | 21.0±3.7 | 36.7±5.3 | 33.8±7.1 | 30.5±4.0 | 17.3±5.2 |
| PEG₆-(fentanyl)₂-Am | 13.7±3.6 | 24.6±6.1 | 30.5±4.0 | 24.4±5.1 | 24.4±5.1 | 21.4±5.1 |
| α-6-O-PEG₄-(codeine)₂ | 14.2±2.3 | 16.7±2.1 | 28.1±1.2 | 30.1±2.1 | 26.4±4.3 | 16.5±3.1 |
| α-6-O-PEG₆-(codeine)₂ | 14.5±3.1 | 15.5±1.8 | 26.3±3.1 | 32.4±1.9 | 27.3±2.3 | 15.3±2.1 |
| α-6-O-PEG₄-(oxycodol)₂ | 15.9±3.7 | 17.3±5.2 | 26.7±5.3 | 25.8±7.1 | 18.7±3.2 | 15.7±4.7 |
| α-6-O-PEG₆-(oxycodol)₂ | 13.8±2.0 | 19.9±4.3 | 24.5±4.0 | 24.4±5.1 | 17.8±5.8 | 18.2±5.8 |

The experimental results showed that the peak of analgesic intensity of the pegylated opioids decreased to varying degrees relative to the original drugs. However, at the same time, it could be observed that the analgesic duration of the pegylated drugs was significantly longer than that of the original drugs, especially the double-ended test drugs, which still had a certain degree of analgesic effect until 24 hours after administration. The analgesic duration of the single-ended compounds was also extended to a certain extent compared with that of the original drugs, and still had a certain analgesic effect at 6th hour after administration. The original drugs had obvious efficacy at 2nd hour after administration, but generally no analgesic effect when observed at 6th hour.

### 17.3 Rat Self-administration Experiment

### Experimental Method:

The rats were randomly divided into a control group and a model group with 8 in each group. An indwelling catheter was placed into the right jugular vein. All the rats with intrajugular catheter were subjected to self-administration session for 2 hours every day from the 6th day after surgery. The rats were trained to nose-poke for drug injection by a fix-ratio-1 (FR1) schedule. The model group (given morphine, oxycodone, and codeine) was injected with a solution with a concentration of 15 mg/mL (50 µL/time, i.e., 0.75 mg/dose/injection), and the control group was injected with normal saline (50 µL/time). Three doses were tested for each test drug, i.e., 0.75 mg/dose/injection, 7.5 mg/dose/injection and 75 mg/dose/injection. The number of times of nose-poke on the 11th day of the experiment was used as an index to evaluate the risk of addiction to each drug. The experimental results are shown in Table 6.

**Table 6**

| Groups | Dose (mg/dose/injection) | Nose-pokes |
|---|---|---|
| NS | - | 2.7±0.7 |
| Morphine | 0.75 | 24.0±2.2 |
| Fentanyl | 0.075 | 19.7±2.1 |
| α-6-O-mPEG₄-morphine | 0.75 | 9.2±0.6 |
| | 7.5 | 11.1±0.4 |
| | 75 | 12.5±0.3 |
| α-6-O-mPEG₆-morphine | 0.75 | 10.3±0.2 |
| | 7.5 | 11.3±0.6 |
| | 75 | 13.8±0.7 |
| α-6-O-PEG₄-(morphine)₂ | 0.75 | 4.5±0.2 |
| | 7.5 | 5.1±0.4 |
| | 75 | 6.3±1.1 |
| α-6-O-PEG₆-(morphine)₂ | 0.75 | 6.7±0.6 |
| | 7.5 | 5.9±0.5 |
| | 75 | 6.3±0.4 |
| PEG₄-(fentanyl)₂-Am | 0.075 | 3.1±0.9 |
| | 0.75 | 5.2±1.1 |
| | 7.5 | 4.9±2.1 |
| PEG₆-(fentanyl)₂-Am | 0.075 | 2.1±0.5 |
| | 0.75 | 3.5±1.1 |
| | 7.5 | 5.7±0.9 |

The experimental results confirmed that the risk of addiction to both morphine and fentanyl decreased significantly after pegylation, and the number of self-administration in rats were significantly reduced compared with the original drugs within the dose range tested. The numbers of self-administration of the original drugs were much more than 100 times the dose of pegylated form of the drugs. Compared with single-ended compound, the double-ended pegylated morphine had a more pronounced decrease in addiction, and the number of self-administration similar to that of the saline group within the dose range tested.

## Claims

1. A conjugate of polyethylene glycol and opioid having a structure of formula (I):
PEG-(X-OP)ₘ (I)
wherein,
PEG is a double-ended, branched or multi-armed polyethylene glycol residue with a molecular weight of 45-1500 Da;
X is a linking group selected from one of the following group: -O-, -OC(O)-, -O(CH₂)ᵢNH-, -O(CH₂)ᵢC(O)O-, -O(CH₂)ᵢC(O)NH-, and -O(CH₂)ᵢNHC(O)NH-, i is an integer of 0-5, preferably 0, 1, or 2;
OP is opioid; and
m is an integer of 2-6, preferably 2, 3 or 4;
wherein the opioid is morphine, hydromorphone, oxycodone, hydrocodone, codeine, paracodin, nalbuphine, methofuran, levorphan, levorphanol, butorphanol, oxymorphone, buprenorphine, or fentanyl;
and, with the proviso that when the PEG is a double-ended polyethylene glycol residue of formula -(CH₂CH₂O)ₙ₋₁CH₂CH₂- and the opioid is morphine, then n is an integer of 6-30.

2. The conjugate of polyethylene glycol and opioid of claim 1, wherein the linking group X is -O-, and the conjugate has a structure of formula (I-0):
PEG-(O-OP)ₘ (I-0).

3. The conjugate of polyethylene glycol and opioid of claim 1 or 2, wherein the PEG is a double-ended polyethylene glycol residue: -(CH₂CH₂O)ₙ₋₁CH₂CH₂-, and n is an integer of 2-30.

4. The conjugate of polyethylene glycol and opioid of claim 1 or 2, wherein the PEG is a double-ended polyethylene glycol residue: -(CH₂CH₂O)ₙ₋₁CH₂CH₂-, and n is 2, 3, 4, 5, 6, 7, 8, 9, or 10.

5. The conjugate of polyethylene glycol and opioid of claim 3 or 4, wherein the conjugate has a structure of the following formulae (IV-1) to (IV-9):

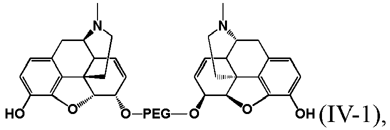

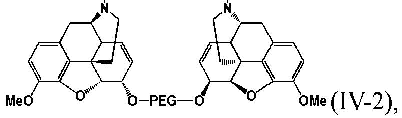

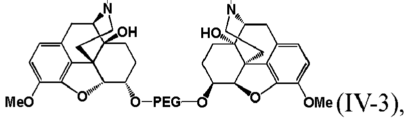

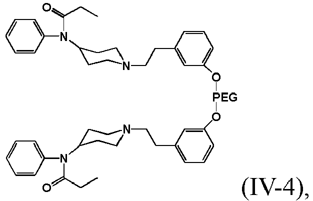

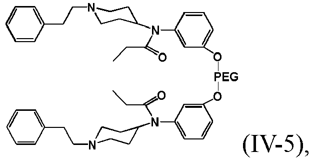

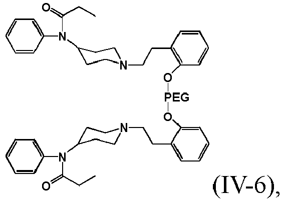

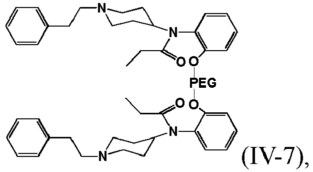

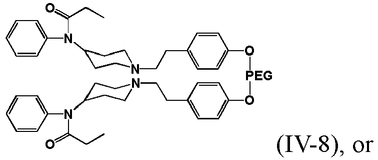

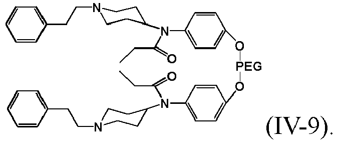

6. The conjugate of polyethylene glycol and opioid of claim 1 or 2, wherein the PEG is a three-armed polyethylene glycol residue represented by the following formula: 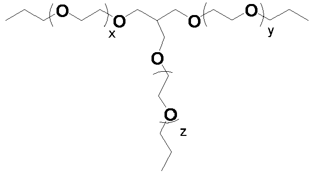 wherein, x, y, or z are independently selected from an integer of 1-20, preferably, an integer of 1-10, and more preferably 1, 2, 3, 4, 5, or 6.

7. The conjugate of polyethylene glycol and opioid of claim 1 or 2, wherein the PEG is a four-armed polyethylene glycol residue represented by the following formula: 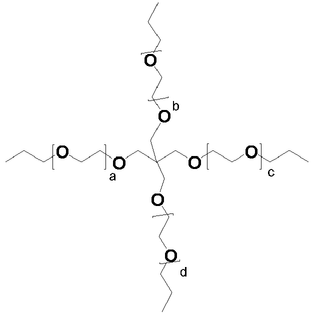 wherein, a, b, c or d are independently selected from the integer of 1-20, preferably, an integer of 1-10, and more preferably 1, 2, 3, 4, 5, or 6.

8. A pharmaceutically acceptable salt of the conjugate of polyethylene glycol and opioid of formula (I) as defined in any one of claims 1-7 selected from the group consisting of: sodium salt, potassium salt, cesium salt, calcium salt, magnesium salt, triethylamine salt, pyridinium salt, picoline salt, ethanolamine salt, triethanolamine salt, dicyclohexylamine salt, N,N-dibenzylethylenediamine salt, hydrochloride, hydrobromide, sulfate, nitrate, phosphate, formate, acetate, trifluoroacetate, citrate, tartrate, fumarate, maleate, lactate, mesylate, benzene sulfonate, tosilate, arginine, aspartate, glutamate, pantothenate, ascorbate, and a combination thereof.

9. A pharmaceutical composition comprising a conjugate of polyethylene glycol and opioid having a structure of formula (I):
PEG-(X-OP)ₘ (I)
wherein,
PEG is a double-ended, branched or multi-armed polyethylene glycol residue with a molecular weight of 45-1500 Da;
X is a linking group selected from one of the following group: -O-, -OC(O)-, -O(CH₂)ᵢNH-, -O(CH₂)ᵢC(O)O-, -O(CH₂)ᵢC(O)NH-, and -O(CH₂)ᵢNHC(O)NH-, i is an integer of 0-5, preferably 0, 1, or 2;
OP is opioid; and
m is an integer of 2-6, preferably 2, 3 or 4;
wherein the opioid is morphine, hydromorphone, oxycodone, hydrocodone, codeine, paracodin, nalbuphine, methofuran, levorphan, levorphanol, butorphanol, oxymorphone, buprenorphine, or fentanyl
and, with the proviso that when the PEG is a double-ended polyethylene glycol residue of formula -(CH₂CH₂O)ₙ₋₁CH₂CH₂- and the opioid is morphine, then n is an integer of 6-30;
or a pharmaceutically acceptable salt thereof,
and a pharmaceutically acceptable carrier or excipient.

10. A conjugate of polyethylene glycol and opioid having a structure of formula (I):
PEG-(X-OP)ₘ (I)
wherein,
PEG is a double-ended, branched or multi-armed polyethylene glycol residue with a molecular weight of 45-1500 Da;
X is a linking group selected from one of the following group: -O-, -OC(O)-, -O(CH₂)ᵢNH-, -O(CH₂)ᵢC(O)O-, -O(CH₂)ᵢC(O)NH-, and -O(CH₂)ᵢNHC(O)NH-, i is an integer of 0-5, preferably 0, 1, or 2;
OP is opioid; and
m is an integer of 2-6, preferably 2, 3 or 4;
wherein the opioid is morphine, hydromorphone, oxycodone, hydrocodone, codeine, paracodin, nalbuphine, methofuran, levorphan, levorphanol, butorphanol, oxymorphone, buprenorphine, or fentanyl and, with the proviso that when the PEG is a double-ended polyethylene glycol residue of formula -(CH₂CH₂O)ₙ₋₁CH₂CH₂- and the opioid is morphine, then n is an integer of 6-30;
or a pharmaceutically acceptable salt thereof,
or the pharmaceutical composition of claim 9,
for use as an analgesic drug.

11. The conjugate of polyethylene glycol and opioid, or the pharmaceutically acceptable salt thereof, or the pharmaceutical composition for use of claim 10, wherein the drug has a reduced addiction.

## Patentansprüche

1. Konjugat aus Polyethylenglykol und Opioid mit einer Struktur der Formel (I):
PEG-(X-OP)ₘ (I)
wobei
PEG ein doppelendiger, verzweigter oder mehrarmiger Polyethylenglykolrest mit einem Molekulargewicht von 45 - 1500 Da ist;
X eine Verbindungsgruppe ist, die aus einer der folgenden Gruppen ausgewählt ist: -O-, -OC(O)-, -O(CH₂)ᵢNH-, -O(CH₂)ᵢC(O)O-, -O(CH₂)ᵢC(O)NH- und - O(CH₂)ᵢNHC(O)NH-, i eine ganze Zahl von 0-5, vorzugsweise 0, 1 oder 2 ist;
OP ein Opioid ist; und
m eine ganze Zahl von 2 - 6, vorzugsweise 2, 3 oder 4 ist;
wobei das Opioid Morphin, Hydromorphon, Oxycodon, Hydrocodon, Codein, Paracodin, Nalbuphin, Methofuran, Levorphan, Levorphanol, Butorphanol, Oxymorphon, Buprenorphin oder Fentanyl ist;
und mit der Maßgabe, dass, wenn das PEG ein Polyethylenglykolrest mit zwei Enden der Formel -(CH₂CH₂O)ₙ₋₁CH₂CH₂- ist und das Opioid Morphin ist, n eine ganze Zahl von 6 - 30 ist.

2. Konjugat aus Polyethylenglykol und Opioid nach Anspruch 1, wobei die Verbindungsgruppe X -O- ist und das Konjugat eine Struktur der Formel (I-0) aufweist:
PEG-(O-OP)ₘ (I-0).

3. Konjugat aus Polyethylenglykol und Opioid nach Anspruch 1 oder 2, wobei das PEG ein Polyethylenglykolrest mit zwei Enden ist: -(CH₂CH₂O)ₙ₋₁CH₂CH₂-, und n eine ganze Zahl von 2 - 30 ist.

4. Konjugat aus Polyethylenglykol und Opioid nach Anspruch 1 oder 2, wobei das PEG ein Polyethylenglykolrest mit zwei Enden ist: -(CH₂CH₂O)ₙ₋₁CH₂CH₂-, und n 2, 3, 4, 5, 6, 7, 8, 9 oder 10 ist.

5. Konjugat aus Polyethylenglykol und Opioid nach Anspruch 3 oder 4, wobei das Konjugat eine Struktur der folgenden Formeln (IV-1) bis (IV-9) aufweist:

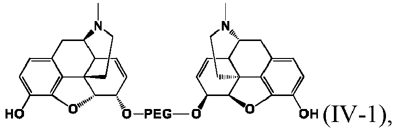

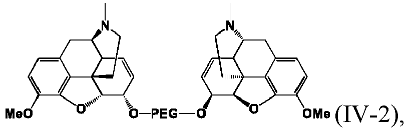

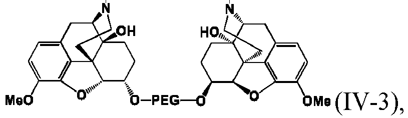

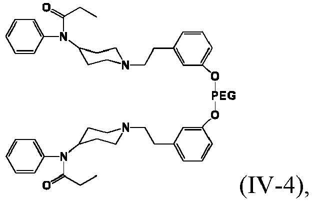

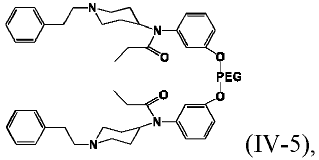

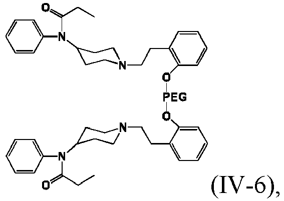

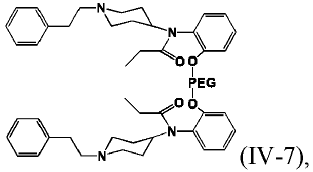

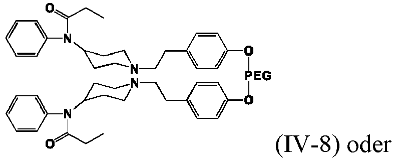

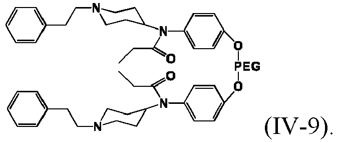

6. Konjugat von Polyethylenglykol und Opioid nach Anspruch 1 oder 2, wobei das PEG ein dreiarmiger Polyethylenglykolrest ist, der durch die folgende Formel dargestellt wird: 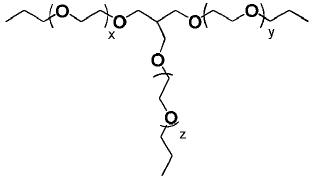 wobei x, y oder z unabhängig voneinander aus einer ganzen Zahl von 1 - 20, vorzugsweise aus einer ganzen Zahl von 1 - 10 und noch bevorzugter aus 1, 2, 3, 4, 5 oder 6 ausgewählt sind.

7. Konjugat von Polyethylenglykol und Opioid nach Anspruch 1 oder 2, wobei das PEG ein vierarmiger Polyethylenglykolrest ist, der durch die folgende Formel dargestellt wird: 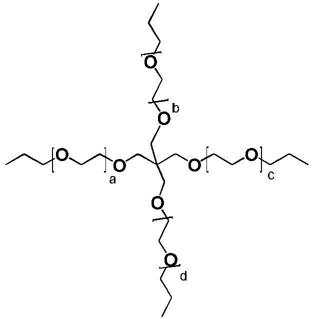 wobei a, b, c oder d unabhängig voneinander aus der ganzen Zahl von 1 - 20, vorzugsweise einer ganzen Zahl von 1 - 10 ausgewählt sind, und noch bevorzugter 1, 2, 3, 4, 5 oder 6 sind.

8. Pharmazeutisch annehmbares Salz des Konjugats von Polyethylenglykol und Opioid der Formel (I), wie in einem der Ansprüche 1 - 7 definiert, ausgewählt aus der Gruppe bestehend aus: Natriumsalz, Kaliumsalz, Cäsiumsalz, Calciumsalz, Magnesiumsalz, Triethylaminsalz, Pyridiniumsalz, Picolinsalz, Ethanolaminsalz, Triethanolaminsalz, Dicyclohexylaminsalz, N,N-Dibenzylethylendiaminsalz, Hydrochlorid, Hydrobromid, Sulfat, Nitrat, Phosphat, Formiat, Acetat, Trifluoracetat, Citrat, Tartrat, Fumarat, Maleat, Lactat, Mesylat, Benzolsulfonat, Tosilat, Arginin, Aspartat, Glutamat, Pantothenat, Ascorbat und eine Kombination davon.

9. Pharmazeutische Zusammensetzung, umfassend ein Konjugat aus Polyethylenglykol und Opioid mit einer Struktur der Formel (I):
PEG-(X-OP)ₘ (I)
wobei,
PEG ein doppelendiger, verzweigter oder mehrarmiger Polyethylenglykolrest mit einem Molekulargewicht von 45 - 1500 Da ist;
X eine Verbindungsgruppe ist, die aus einer der folgenden Gruppen ausgewählt ist: -O-, -OC(O)-, -O(CH₂)ᵢNH-, -O(CH₂)ᵢC(O)O-, -O(CH₂)ᵢC(O)NH- und - O(CH₂)ᵢNHC(O)NH-, i eine ganze Zahl von 0 - 5, vorzugsweise 0, 1 oder 2, ist;
OP ein Opioid ist; und
m eine ganze Zahl von 2 - 6, vorzugsweise 2, 3 oder 4 ist;
wobei das Opioid Morphin, Hydromorphon, Oxycodon, Hydrocodon, Codein, Paracodin, Nalbuphin, Methofuran, Levorphan, Levorphanol, Butorphanol, Oxymorphon, Buprenorphin oder Fentanyl ist;
und mit der Maßgabe, dass, wenn das PEG ein Polyethylenglykolrest mit zwei Enden der Formel -(CH₂CH₂O)ₙ₋₁CH₂CH₂- ist und das Opiod Morphin ist, n eine ganze Zahl von 6 - 30 ist;
oder ein pharmazeutisch annehmbares Salz davon,
und einen pharmazeutisch annehmbaren Träger oder Hilfsstoff.

10. Konjugat aus Polyethylenglykol und Opioid mit einer Struktur der Formel (I):
PEG-(X-OP)ₘ (I)
wobei
PEG ein doppelendiger, verzweigter oder mehrarmiger Polyethylenglykolrest mit einem Molekulargewicht von 45 - 1500 Da ist;
X eine Verbindungsgruppe ist, die aus einer der folgenden Gruppen ausgewählt ist: -O-, -OC(O)-, -O(CH₂)ᵢNH-, -O(CH₂)ᵢC(O)O-, -O(CH₂)ᵢC(O)NH- und - O(CH₂)ᵢNHC(O)NH-, i eine ganze Zahl von 0 - 5, vorzugsweise 0, 1 oder 2 ist;
OP ein Opioid ist; und
m eine ganze Zahl von 2 - 6, vorzugsweise 2, 3 oder 4 ist;
wobei das Opioid Morphin, Hydromorphon, Oxycodon, Hydrocodon, Codein, Paracodin, Nalbuphin, Methofuran, Levorphan, Levorphanol, Butorphanol, Oxymorphon, Buprenorphin oder Fentanyl ist;
und mit der Maßgabe, dass, wenn das PEG ein Polyethylenglykolrest mit zwei Enden der Formel -(CH₂CH₂O)ₙ₋₁CH₂CH₂- ist und das Opiod Morphin ist, n eine ganze Zahl von 6 - 30 ist;
oder ein pharmazeutisch annehmbares Salz davon,
oder die pharmazeutische Zusammensetzung nach Anspruch 9,
zur Verwendung als Analgetikum.

11. Das Konjugat von Polyethylenglykol und Opioid oder das pharmazeutisch annehmbare Salz davon oder die pharmazeutische Zusammensetzung zur Verwendung nach Anspruch 10, wobei das Medikament eine reduzierte Abhängigkeit aufweist.

## Revendications

1. Conjugué de polyéthylène glycol et d'opioïde ayant une structure de formule (I) :
PEG-(X-OP)ₘ (I)
où,
PEG est un résidu de polyéthylène glycol à deux extrémités, ramifié ou à plusieurs bras, ayant une masse moléculaire de 45-1500 Da ;
X est un groupe de liaison choisi parmi l'un des groupes suivants : -O-, -OC(O)-, -O(CH₂)ᵢNH-, -O(CH₂)ᵢC(O)O-, -O(CH₂)ᵢC(O)NH-, et -O(CH₂)ᵢNHC(O)NH-, i est un nombre entier de 0-5, de préférence 0, 1, ou 2 ;
OP est un opioïde ; et
m est un nombre entier de 2-6, de préférence 2, 3 ou 4 ;
où l'opioïde est la morphine, l'hydromorphone, l'oxycodone, l'hydrocodone, la codéine, la paracodine, la nalbuphine, le méthofurane, le lévorphan, le lévorphanol, le butorphanol, l'oxymorphone, la buprénorphine, ou le fentanyl ;
et, à condition que lorsque le PEG est un résidu de polyéthylène glycol à deux extrémités de formule -(CH₂CH₂O)ₙ₋₁CH₂CH₂- et que l'opioïde est la morphine, alors n est un nombre entier de 6-30.

2. Conjugué de polyéthylène glycol et d'opioïde selon la revendication 1, où le groupe de liaison X est -O-, et le conjugué à une structure de formule (I-0) :
PEG-(O-OP)ₘ (I-0).

3. Conjugué de polyéthylène glycol et d'opioïde selon la revendication 1 ou 2, où le PEG est un résidu de polyéthylène glycol à deux extrémités : -(CH₂CH₂O)ₙ₋₁CH₂CH₂-, et n est un nombre entier de 2-30.

4. Conjugué de polyéthylène glycol et d'opioïde selon la revendication 1 ou 2, où le PEG est un résidu de polyéthylène glycol à deux extrémités : -(CH₂CH₂O)ₙ₋₁CH₂CH₂-, et n vaut 2, 3, 4, 5, 6, 7, 8, 9, ou 10.

5. Conjugué de polyéthylène glycol et d'opioïde selon la revendication 3 ou 4, où le conjugué a une structure des formules (IV-I) à (IV-9) suivantes :

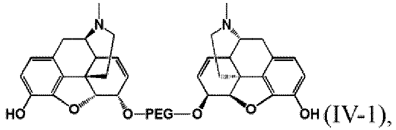

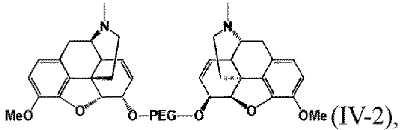

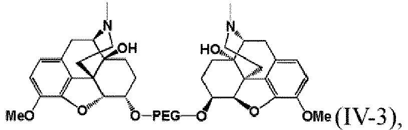

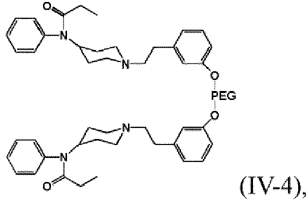

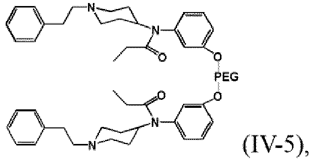

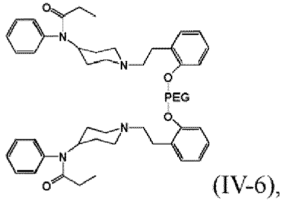

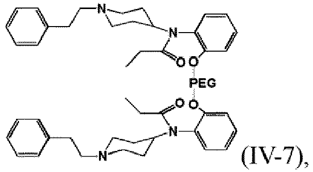

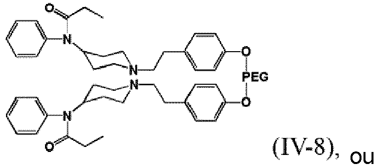

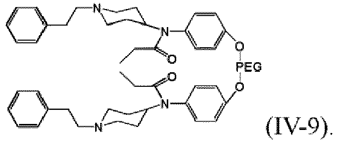

6. Conjugué de polyéthylène glycol et d'opioïde selon la revendication 1 ou 2, où le PEG est un résidu de polyéthylène glycol à trois bras représenté par la formule suivante : 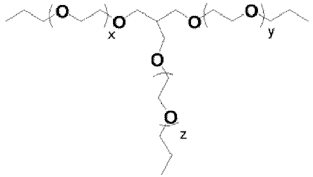 où x, y, ou z sont indépendamment choisis parmi un nombre entier de 1-20, de préférence un nombre entier de 1-10, et plus préférablement 1, 2, 3, 4, 5, ou 6.

7. Conjugué de polyéthylène glycol et d'opioïde selon la revendication 1 ou 2, où le PEG est un résidu de polyéthylène glycol à quatre bras représenté par la formule suivante : 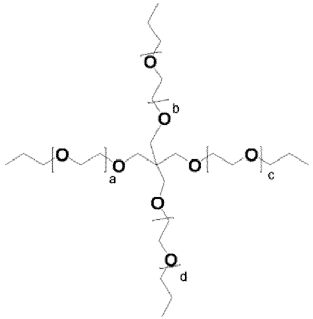 où a, b, c ou d sont indépendamment choisis parmi les nombres entiers de 1-20, de préférence un nombre entier de 1-10, et plus préférablement 1, 2, 3, 4, 5, ou 6.

8. Sel pharmaceutiquement acceptable du conjugué de polyéthylène glycol et d'opioïde de formule (I) tel que défini dans l'une quelconque des revendications 1-7, choisi dans le groupe constitué par : sel de sodium, sel de potassium, sel de césium, sel de calcium, sel de magnésium, sel de triéthylamine, sel de pyridinium, sel de picoline, sel d'éthanolamine, sel de triéthanolamine, sel de dicyclohexylamine, sel de N,N-dibenzyléthylènediamine, chlorhydrate, bromhydrate, sulfate, nitrate, phosphate, formiate, acétate, trifluoroacétate, citrate, tartrate, fumarate, maléate, lactate, mésylate, benzènesulfonate, tosilate, arginine, aspartate, glutamate, pantothénate, ascorbate, et une combinaison de ceux-ci.

9. Composition pharmaceutique comprenant un conjugué de polyéthylène glycol et d'opioïde ayant une structure de formule (I) :
PEG-(X-OP)ₘ (I)
où,
PEG est un résidu de polyéthylène glycol à deux extrémités, ramifié ou à plusieurs bras, ayant une masse moléculaire de 45-1500 Da ;
X est un groupe de liaison choisi parmi l'un des groupes suivants : -O-, -OC(O)-, -O(CH₂)ᵢNH-, -O(CH₂)ᵢC(O)O-, -O(CH₂)ᵢC(O)NH-, et -O(CH₂)ᵢNHC(O)NH-, i est un nombre entier de 0-5, de préférence 0, 1, ou 2 ;
OP est un opioïde ; et
m est un nombre entier de 2-6, de préférence 2, 3 ou 4 ;
où l'opioïde est la morphine, l'hydromorphone, l'oxycodone, l'hydrocodone, la codéine, la paracodine, la nalbuphine, le méthofurane, le lévorphan, le lévorphanol, le butorphanol, l'oxymorphone, la buprénorphine, ou le fentanyl et, à condition que lorsque le PEG est un résidu de polyéthylène glycol à deux extrémités de formule -(CH₂CH₂O)ₙ₋₁CH₂CH₂- et que l'opioïde est la morphine, alors n est un nombre entier de 6-30 ;
ou un sel pharmaceutiquement acceptable de celui-ci,
et un support ou excipient pharmaceutiquement acceptable.

10. Conjugué de polyéthylène glycol et d'opioïde ayant une structure de formule (I) :
PEG-(X-OP)ₘ (I)
où,
PEG est un résidu de polyéthylène glycol à deux extrémités, ramifié ou à plusieurs bras, ayant une masse moléculaire de 45-1500 Da ;
X est un groupe de liaison choisi parmi l'un des groupes suivants : -O-, -OC(O)-, -O(CH₂)ᵢNH-, -O(CH₂)ᵢC(O)O-, -O(CH₂)ᵢC(O)NH-, et -O(CH₂)ᵢNHC(O)NH-, i est un nombre entier de 0-5, de préférence 0, 1, ou 2 ;
OP est un opioïde ; et
m est un nombre entier de 2-6, de préférence 2, 3 ou 4 ;
où l'opioïde est la morphine, l'hydromorphone, l'oxycodone, l'hydrocodone, la codéine, la paracodine, la nalbuphine, le méthofurane, le lévorphan, le lévorphanol, le butorphanol, l'oxymorphone, la buprénorphine, ou le fentanyl et, à condition que lorsque le PEG est un résidu de polyéthylène glycol à deux extrémités de formule -(CH₂CH₂O)ₙ₋₁CH₂CH₂- et que l'opioïde est la morphine, alors n est un nombre entier de 6-30 ;
ou un sel pharmaceutiquement acceptable de celui-ci,
ou la composition pharmaceutique selon la revendication 9,
pour une utilisation comme médicament analgésique.

11. Conjugué de polyéthylène glycol et d'opioïde, ou sel pharmaceutiquement acceptable de celui-ci, ou composition pharmaceutique pour une utilisation selon la revendication 10, où le médicament a une dépendance réduite.
